# EUROPEAN PATENT APPLICATION

(11) **EP 4 635 980 A1**
(43) Date of publication of application: **22.10.2025**
(21) Application number: 24171403.9
(22) Date of filing: 19.04.2024
(51) Int. Cl.: C07K 16/00, C07K 16/28

(54) **UNI-TAGS SPECIFIC ANTIBODY**

(71) Applicant: Medigene Immunotherapies GmbH, 82152 Planegg-Martinsried (DE)
(72) Inventor: The designation of the inventor has not yet been filed
(74) Representative: Maiwald GmbH

(57) **Abstract**

The present invention relates to an antibody or binding fragment thereof that binds to a protein, preferably a T cell receptor, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V). Further, the invention relates to pharmaceutical compositions comprising such an antibody or binding fragment thereof and to the use of such an antibody or binding fragment thereof for use as a medicament or for use as a screening agent.

## Description

### FIELD OF THE INVENTION

The present invention relates to an antibody or binding fragment thereof that binds to a protein, preferably a T cell receptor, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V). Further, the invention relates to pharmaceutical compositions comprising such an antibody or binding fragment thereof and to the use of such an antibody or binding fragment thereof for use as a medicament or for use as a screening agent.

### BACKGROUND OF THE INVENTION

In the realm of research and development, epitope-tagged proteins enable precise visualization and tracking of recombinant proteins, facilitating a deeper understanding of their behavior and functionality.

The number of available tags in the human system is limited, and even more narrowed when only linear epitopes are counted. The number of linear epitopes suggested to be non-immunogenic is even smaller.

Hence there is a great need for further epitope tags in particular for linear epitope tags to be included into proteins expressed in the human system.

As an example, recombinant epitope-tagged TCRs (rTCRs) are used.

In the context of clinical adoptive immune therapy, epitope-tagged rTCRs provide significant benefits, allowing for the visualization and isolation of specific T-cell populations, thereby greatly enhancing the precision and efficacy of therapeutic interventions. This technology enables the identification and enrichment of highly functional T cells that possess the ability to recognize and eliminate cancer cells or pathogens. Epitope-tagged rTCRs are also useful for drug product characterization and release. Additionally, epitope-tagged rTCRs offer the potential for real-time monitoring of T-cell localization and persistence *in vivo,* optimizing treatment protocols and improving patient outcomes. In addition to their primary applications, epitope-tagged rTCRs hold the potential to serve as a vital safety switch in adoptive immune therapy. They offer a promising avenue for the depletion of malfunctional or overly potent T cells that have been transferred to patients. Particularly, epitope-tagged rTCRs may eventually function as a safeguard, allowing clinicians to selectively remove undesirable T-cell populations, thereby minimizing the risk of adverse effects and optimizing the overall safety and efficacy of adoptive T-cell therapies. Furthermore, epitope-tagged rTCRs of different sequence can be compared in a standardized manner to assess potential differences in surface expression, isolation of enriched populations of immune cells expressing rTCRs that can be compared with each other.

Overall, the application of epitope-tagged proteins, such as epitope-tagged rTCRs, such as in adoptive immune therapy, represents a powerful approach that combines both research insights and clinical benefits. By leveraging the capabilities of such tagged receptors, researchers and clinicians can advance understanding behavior of immune cells expressing rTCRs, including T cells and natural killer cells, and unlock new opportunities for targeted and personalized therapeutic interventions. In this context, a particular challenge is to identify an epitope that is not immunogenic itself.

Antibodies binding to specific proteins can be powerful tools, offering a versatile and biologically compatible platform for protein tagging and/or manipulation across diverse medical and research domains. For instance, epitope-tagged rTCRs allow for visualization and/or elimination of (unwanted) T cells from a patient.

There is thus a need for new epitopes that are not immunogenic itself and antibodies which allow for the targeting of the specific new epitopes. For example, there is a need for antibodies which allow for the targeting of specific populations of rTCRs and thus specific populations of immune cells expressing rTCRs.

### OBJECTIVES AND SUMMARY OF THE INVENTION

Against this background, it is one objective of the present invention to provide new tags as well as antibodies or fragments thereof which bind and/or detect specific populations of epitope-tagged proteins.

It is another objective of the present invention to provide antibodies or fragments thereof for use in therapy and/or analytical scenarios.

As surprisingly discovered by the present inventors, said objectives are realized by antibodies as herein disclosed and defined, which allow for the binding and/or detecting of specific populations of epitope-tagged proteins.

The inventors herewith present a new epitope tag that can be used to tag any expressed protein. The provided tag can be used for visualization, enrichment, characterization and purification of the protein to be tagged. In particular for membrane proteins, the tag can be used for visualization, enrichment, characterization of the cell type in which the tagged protein is integrated and further could be used as a safety switch. The provided tag provides excellent non-immungenic characteristics and the sequence is unique and not present in the human body. The UNI-Tag can flexible be inserted internal, C-terminal or N-terminal of the desired sequence.

The provided epitope sequence significantly diminishes the likelihood of eliciting immune responses when the epitope is introduced in a protein of interest, ensuring heightened safety in patient applications. A linearly structured epitope when used as a tag maintains the structural integrity and functional capabilities of the tagged proteins, preserving their native properties and activities.

As long as the epitope is accessible by the according antibody, the UNI-Tag serves as useful tagging tool in the natural configuration. As linear epitope it can be used also at positions which are not accessible in the natural configuration but are accessible in denatured proteins.

In particular it is one objective of the present invention to provide new tags as well as antibodies or fragments thereof which bind and/or detect specific populations of epitope-tagged proteins, such as rTCRs and thus specific populations of immune cells expressing rTCRs.

Exemplary said objectives are realized by epitope tags and corresponding antibodies as herein disclosed and defined, which allow for the binding and/or detecting of specific populations of epitope-tagged proteins, such as TCRs, such as rTCRs.

As surprisingly discovered, the respective "UNI-tag epitope", as herein disclosed and defined, can be used to tag any expressed protein, either inserted internal, C-terminal or N-terminal of the desired sequence. As long as the epitope is accessible by the antibody according to the present invention, the UNI-tag serves as highly useful tagging tool. As furthermore surprisingly discovered, the UNI-tag epitope is non-immunogenic, i.e., it does not provoke an immune response in the human and/or animal body. The number of available tags in the human system is limited, and even more narrowed when only linear epitopes are counted. The number of linear epitopes suggested to be non-immunogenic is even smaller. For all these reasons, the herein disclosed UNI-tag system constitutes an outstanding invention. The epitope is the sequence detected by the antibody in an antigen, which is part of or corresponds to the recombinant (tagged) protein.

Consequently, in a first aspect, the present invention provides an antibody or binding fragment thereof that binds to a protein, preferably a T cell receptor, such as a recombinant T cell receptor, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V)

In some embodiments, the antibody or binding fragment thereof binds to a protein, preferably the T cell receptor, comprising an epitope comprising or consisting of an amino acid sequence having at least 85 %, such as 88 %, more preferably at least 95 %, for instance 100 % sequence identity to the amino acid sequence set forth in SEQ ID NO: 63 (GEVPKX₁RFS), wherein X₁ is as defined herein above.

In other embodiments, the antibody or binding fragment thereof binds to a protein, preferably T cell receptor, comprising an epitope comprising or consisting of an amino acid sequence set forth in SEQ ID NO: 64 (X₂X₃GEVPKX₁RFSX₄), wherein X₁ is as defined herein above; X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

In another embodiment, the antibody or binding fragment thereof binds to a protein, preferably T cell receptor, comprising an epitope comprising or consisting of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

In some embodiments, the antibody or binding fragment thereof belongs to the rat IgG2a subtype, preferably comprising a kappa light chain.

In a further embodiment, the antibody or binding fragment thereof is capable of tagging a protein, preferably a TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

In another embodiment, the antibody or binding fragment thereof comprises a light chain variable region and/or a heavy chain variable region, wherein the light chain variable region comprises at least a CDR1 set forth in SEQ ID NO: 3 or sequences at least 80% identical thereto, a CDR2 set forth in SEQ ID NO: 4 or sequences at least 80% identical thereto, and/or a CDR3 set forth in SEQ ID NO: 5 or sequences at least 80% identical thereto; and/or wherein the heavy chain variable region comprises at least a CDR1 set forth in SEQ ID NO: 7 or sequences at least 80% identical thereto, a CDR2 set forth in SEQ ID NO: 8 or sequences at least 80% identical thereto, and/or a CDR3 set forth in SEQ ID NO: 9 or sequences at least 80% identical thereto.

In still another embodiment, the antibody or binding fragment thereof comprises a light chain variable region and a heavy chain variable region, wherein the light chain variable region comprises at least a CDR1 set forth in SEQ ID NO: 3, a CDR2 set forth in SEQ ID NO: 4, a CDR3 set forth in SEQ ID NO: 5; and wherein the heavy chain variable region comprises at least a CDR1 set forth in SEQ ID NO: 7, a CDR2 set forth in SEQ ID NO: 8, and/or a CDR3 set forth in SEQ ID NO: 9.

In yet another embodiment, the antibody or binding fragment thereof comprises a light chain comprising or consisting of SEQ ID NO: 2 or sequences at least 80% identical thereto and/or a heavy chain comprising or consisting of SEQ ID NO: 6 or sequences at least 80% identical thereto.

In some embodiments, the antibody or binding fragment thereof comprises a light chain comprising or consisting of SEQ ID NO: 2 and/or a heavy chain comprising or consisting of SEQ ID NO: 6.

In some embodiments, the antibody or binding fragment thereof according to the invention is a monoclonal antibody or binding fragment thereof. The antibody may be a chimeric, murine, humanized or human antibody or binding fragment thereof.

Further, the application in another aspect is concerned with the provision of an antibody or binding fragment thereof as defined herein for use as a medicament or for use as a screening agent.

In particular, the application relates to the provision of an antibody or binding fragment thereof according to the invention for use in the therapy of malignancies, preferably for use in the treatment of cancer, viral diseases, or autoimmunity.

Further, the application relates to the use of an antibody or binding fragment thereof, as defined herein, for tagging a protein, preferably a TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

In a further aspect, the present invention relates to a recombinant protein, characterized in comprising an epitope comprising or consisting of the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

In some embodiments of this aspect, said epitope comprises or consists of an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

### FIGURE LEGENDS

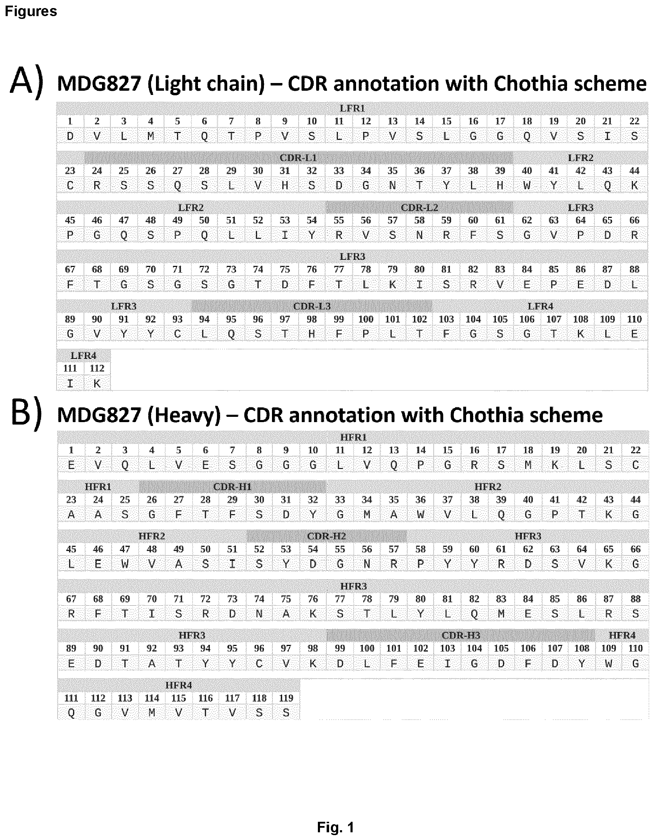

**Figure 1****:** Variable sequence annotation of MDG827 light chain and heavy chain using the Chothia Scheme. Illustrated are the CDRs of both the A) light chain and B) heavy chain of the exemplary MDG827 antibody according to the present invention.

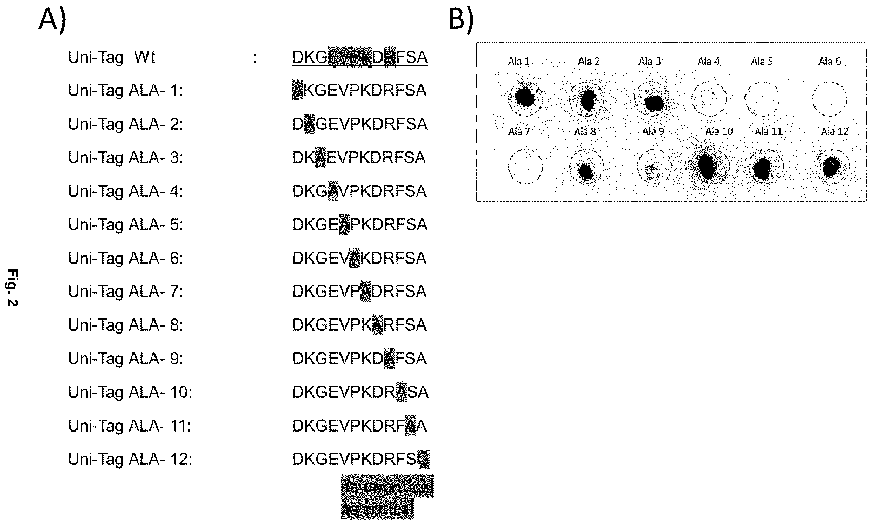

**Figure 2****:** Alanine scanning for MDG827 antibody & epitope interaction. A) Wildtype UNI-tag (SEQ ID NO: 1) & mutagenic UNI-tags (ALA-1 (SEQ ID NO: 18) to ALA-12 (SEQ ID NO: 29) used for alanine scanning, B) Dot blotting was performed to detect mutagenic UNI-tag (ALA-1 (SEQ ID NO: 18) to ALA-12 (SEQ ID NO: 29)) using a MDG827 hybridoma supernatant followed by detection with a horseradish peroxidase-labeled anti-IgG2a secondary antibody. Red & green highlighted aa shows UNI-Tag uncritical aa & critical aa for MDG827 Ab binding respectively.

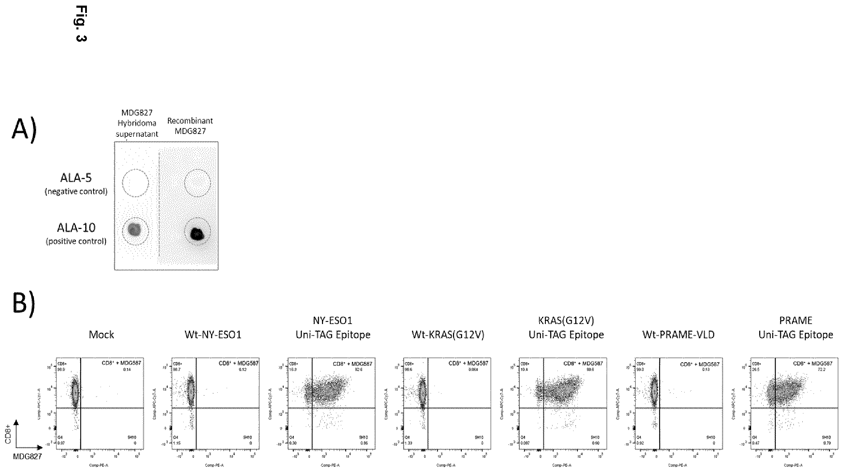

**Figure 3****:** Dot-blot & Jurkat 76 -/-CD8+ ieGFP Cells Staining with Recombinantly Produced MDG827 Targeting UNI-Tag carrying rTCRs. A) Dot blotting was performed to detect mutagenic UNI-Tag (ALA-10 (positive control) and ALA-5 (negative control)) using a recombinantly produced MDG827 primary antibody followed by detection with an HRP-labeled anti-IgG2a secondary antibody. B) Binding analysis of the recombinant monoclonal MDG827 antibody to UNI-tag carrying NY-ESO-1 (T11.8-10-17), PRAME-VLD (T4.8-1-29), or KRAS(G12V)(T47.8-41-071) specific rTCRs in Jurkat 76 -/-CD8+ ieGFP cells. Secondary staining of the MDG827 antibody was performed using an anti-rat IgG2a antibody labeled with PE.

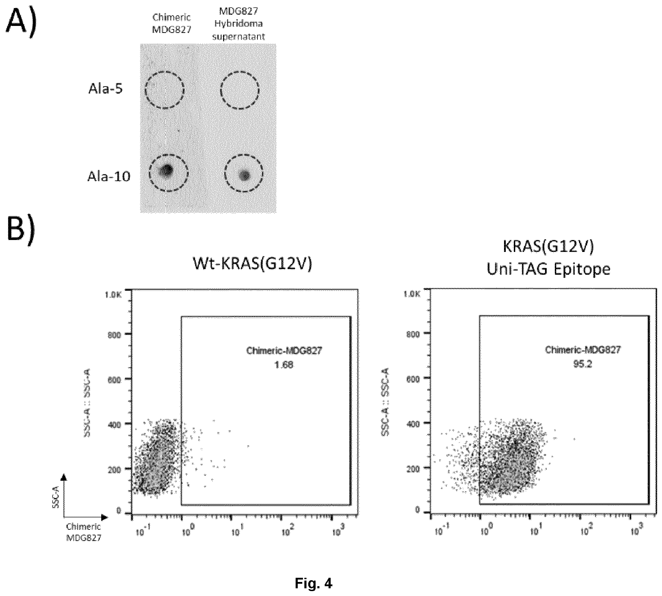

**Figure 4****:** Dot-blot & Jurkat 76 -/-CD8+ ieGFP Cells Staining with recombinantly produced chimeric MDG827 antibody Targeting Uni-tag carrying TCRs. A) Dot blotting was performed to detect mutagenic Uni-tag (ALA-10 (positive control) and ALA-5 (negative control)) using a recombinantly produced chimeric MDG827 primary antibody followed by detection with an HRP-labeled human anti-lgG1 secondary antibody. B) Binding analysis of the recombinantly produced chimeric MDG827 primary antibody to Uni-Tag carrying KRAS(G12V)(T47.8-41-071) specific TCRs at position A199 in loop 1 of TRBC in Jurkat 76 - /-CD8+ ieGFP cells.

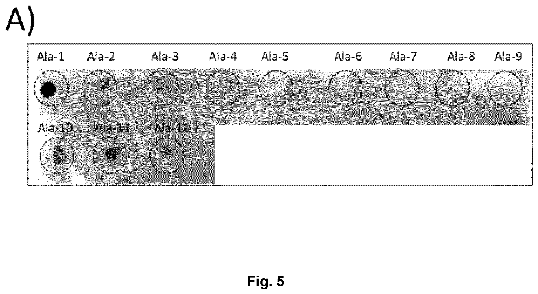

**Figure 5****:** Alanine scanning for humanized MDG827 antibody & epitope interaction. A) Dot blotting was performed to detect mutagenic Uni-Tag (ALA-1 to ALA-12) using a recombinantly expressed humanized MDG827 followed by detection with an HRP-labeled human anti-lgG1 secondary antibody.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures, but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter, e.g., an antibody is defined to be obtainable from a specific source, this is also to be understood to disclose an antibody which is obtained from this source.

Where an indefinite or definite article is used when referring to a singular noun, e.g., "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

One aspect of the invention refers to an antibody or binding fragment thereof that binds to a protein, preferably a T cell receptor, such as a recombinant TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V). Accordingly, in other words, the antibody or binding fragment thereof in accordance with the present invention is capable of binding to an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R).

In more particular embodiments, said epitope comprises or consists of an amino acid sequence having at least 85 %, such as 88 %, more preferably at least 95 %, for instance 100 % sequence identity to the amino acid sequence set forth in SEQ ID NO: 63 (GEVPKX₁RFS), wherein X₁ is as defined herein above.

Furthermore envisaged is that said epitope comprises or consists of an amino acid sequence set forth in SEQ ID NO: 64 (X₂X₃GEVPKX₁RFSX₄), wherein X₁ is as defined herein above; X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

In particular embodiments, said epitope consists of an amino acid sequence set forth in SEQ ID NO: 64 (X₂X₃GEVPKX₁RFSX₄), wherein X₁ is as defined herein above; X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

For instance, in some embodiments, said epitope comprises or consists of an amino acid sequence selected from:
- DX₃GEVPKX₁RFSX₄ (SEQ ID NO: 65);
- EX₃GEVPKX₁RFSX₄ (SEQ ID NO: 66);
- X₂KGEVPKX₁RFSX₄ (SEQ ID NO: 67);
- X₂RGEVPKX₁RFSX₄ (SEQ ID NO: 68);
- X₂HGEVPKX₁RFSX₄ (SEQ ID NO: 69);
- X₂X₃GEVPKX₁RFSG (SEQ ID NO: 70);
- X₂X₃GEVPKX₁RFSL (SEQ ID NO: 71);
- X₂X₃GEVPKX₁RFSA (SEQ ID NO: 72);
- X₂X₃GEVPKX₁RFSV (SEQ ID NO: 73);
- X₂X₃GEVPKX₁RFSM (SEQ ID NO: 74);
- X₂X₃GEVPKX₁RFSI (SEQ ID NO: 75);
- X₂X₃GEVPKX₁RFSS (SEQ ID NO: 76);
- X₂X₃GEVPKX₁RFST (SEQ ID NO: 77),
wherein, where applicable, wherein X₁ is selected from A, R, N, D, C, Q, E, G, H, I, L, K, M, F, P, S, T, W, Y, V; X₂ is selected from D and E; X₃ is selected from K, R, and H; and X₄ is selected from G, L, A, V, M, I, S, and T.

In some embodiments, said epitope comprises or consists of an amino acid sequence selected from:
- DKGEVPKX₁RFSX₄ (SEQ ID NO: 78);
- DRGEVPKX₁RFSX₄(SEQ ID NO: 79);
- DHGEVPKX₁RFSX₄(SEQ ID NO: 80);
- EKGEVPKX₁RFSX₄ (SEQ ID NO: 81);
- ERGEVPKX₁RFSX₄ (SEQ ID NO: 82);
- EHGEVPKX₁RFSX₄ (SEQ ID NO: 83),
wherein X₁ and X₄ are as defined herein above.

In various embodiments, said epitope comprises or consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

In some embodiments, said epitope consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

In various embodiments, said epitope comprises or consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to the amino acid sequence set forth in SEQ ID NO: 1 (DKGEVPKDRFSA).

In various embodiments, said epitope consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to the amino acid sequence set forth in SEQ ID NO: 1 (DKGEVPKDRFSA).

In some embodiments, the epitope, as herein defined, is comprised in a T cell receptor (TCR) and is preferably located within the constant region of the TCR.

In some embodiments, the epitope is not present within CDRs or in the surrounding framework region of the TCR.

The antibody or binding fragment thereof of the present invention comprises, in some embodiments, a light chain variable region and/or a heavy chain variable region.

Particularly, in some embodiments, the antibody or binding fragment thereof is characterized in comprising a light chain variable region comprising at least a CDR1 set forth in SEQ ID NO: 3 or sequences at least 80% identical thereto, a CDR2 set forth in SEQ ID NO: 4 or sequences at least 80% identical thereto, and/or a CDR3 set forth in SEQ ID NO: 5 or sequences at least 80% identical thereto.

In some embodiments, the antibody or binding fragment thereof is characterized in comprising a light chain variable region comprising at least a CDR1 set forth in SEQ ID NO: 3 or sequences at least 80% identical thereto, a CDR2 set forth in SEQ ID NO: 4 or sequences at least 80% identical thereto, and a CDR3 set forth in SEQ ID NO: 5 or sequences at least 80% identical thereto.

In further embodiments, the antibody or binding fragment thereof is characterized in comprising a heavy chain variable region comprising at least a CDR1 set forth in SEQ ID NO: 7 or sequences at least 80% identical thereto, a CDR2 set forth in SEQ ID NO: 8 or sequences at least 80% identical thereto, and/or a CDR3 set forth in SEQ ID NO: 9 or sequences at least 80% identical thereto.

In some embodiments, the antibody or binding fragment thereof is characterized in comprising a heavy chain variable region comprising at least a CDR1 set forth in SEQ ID NO: 7 or sequences at least 80% identical thereto, a CDR2 set forth in SEQ ID NO: 8 or sequences at least 80% identical thereto, and a CDR3 set forth in SEQ ID NO: 9 or sequences at least 80% identical thereto.

In particular embodiments, the antibody or binding fragment thereof is characterized in that the light chain variable region comprises at least a CDR1 set forth in SEQ ID NO: 3 or sequences at least 80% identical thereto, a CDR2 set forth in SEQ ID NO: 4 or sequences at least 80% identical thereto, and/or a CDR3 set forth in SEQ ID NO: 5 or sequences at least 80% identical thereto; and/or in that the heavy chain variable region comprises at least a CDR1 set forth in SEQ ID NO: 7 or sequences at least 80% identical thereto, a CDR2 set forth in SEQ ID NO: 8 or sequences at least 80% identical thereto, and/or a CDR3 set forth in SEQ ID NO: 9 or sequences at least 80% identical thereto.

In some such embodiments, the antibody or binding fragment thereof is characterized in that the light chain variable region comprises at least a CDR1 set forth in SEQ ID NO: 3 or sequences at least 80% identical thereto, a CDR2 set forth in SEQ ID NO: 4 or sequences at least 80% identical thereto, and a CDR3 set forth in SEQ ID NO: 5 or sequences at least 80% identical thereto; and/or in that the heavy chain variable region comprises at least a CDR1 set forth in SEQ ID NO: 7 or sequences at least 80% identical thereto, a CDR2 set forth in SEQ ID NO: 8 or sequences at least 80% identical thereto, and a CDR3 set forth in SEQ ID NO: 9 or sequences at least 80% identical thereto.

In some embodiments, the antibody or binding fragment thereof is characterized in that the light chain variable region comprises at least a CDR1 set forth in SEQ ID NO: 3 or sequences at least 80% identical thereto, a CDR2 set forth in SEQ ID NO: 4 or sequences at least 80% identical thereto, and a CDR3 set forth in SEQ ID NO: 5 or sequences at least 80% identical thereto; and in that the heavy chain variable region comprises at least a CDR1 set forth in SEQ ID NO: 7 or sequences at least 80% identical thereto, a CDR2 set forth in SEQ ID NO: 8 or sequences at least 80% identical thereto, and a CDR3 set forth in SEQ ID NO: 9 or sequences at least 80% identical thereto.

In various embodiments, the antibody or binding fragment thereof is characterized in comprising a light chain comprising or consisting of an amino acid sequence having at least 80 %, preferably at least 85 %, more preferably at least 90 %, even more preferably at least 95 %, such as 100 % sequence identity to the amino acid sequence set forth in SEQ ID NO: 2.

In various embodiments, the antibody or binding fragment thereof is characterized in comprising a heavy chain comprising or consisting of an amino acid sequence having at least 80 %, preferably at least 85 %, more preferably at least 90 %, even more preferably at least 95 %, such as 100 % sequence identity to the amino acid sequence set forth in SEQ ID NO: 6.

In various embodiments, the antibody or binding fragment thereof is characterized in comprising a light chain comprising or consisting of an amino acid sequence having at least 80 %, preferably at least 85 %, more preferably at least 90 %, even more preferably at least 95 %, such as 100 % sequence identity to the amino acid sequence set forth in SEQ ID NO: 2 and a heavy chain comprising or consisting of an amino acid sequence having at least 80 %, preferably at least 85 %, more preferably at least 90 %, even more preferably at least 95 %, such as 100 % sequence identity to the amino acid sequence set forth in SEQ ID NO: 6.

In some embodiments, the antibody or binding fragment thereof is characterized in comprising a light chain consisting of an amino acid sequence having at least 80 %, preferably at least 85 %, more preferably at least 90 %, even more preferably at least 95 %, such as 100 % sequence identity to the amino acid sequence set forth in SEQ ID NO: 2 and a heavy chain consisting of an amino acid sequence having at least 80 %, preferably at least 85 %, more preferably at least 90 %, even more preferably at least 95 %, such as 100 % sequence identity to the amino acid sequence set forth in SEQ ID NO: 6.

The antibody or binding fragment thereof according to the present invention can be further characterized in terms of the nucleotide sequence coding for its amino acid sequence.

Thus, in various embodiments, the antibody or binding fragment thereof is characterized in comprising a light chain variable region comprising at least a CDR1 encoded by the nucleotide sequence set forth in SEQ ID NO: 11 or sequences at least 80% identical thereto, and/or a CDR2 encoded by the nucleotide sequence set forth in SEQ ID NO: 12 or sequences at least 80% identical thereto, and/or a CDR3 encoded by the nucleotide sequence set forth in SEQ ID NO: 13 or sequences at least 80% identical thereto.

In some embodiments, the antibody or binding fragment thereof is characterized in comprising a light chain variable region comprising at least a CDR1 encoded by the nucleotide sequence set forth in SEQ ID NO: 11 or sequences at least 80% identical thereto, a CDR2 encoded by the nucleotide sequence set forth in SEQ ID NO: 12 or sequences at least 80% identical thereto, and a CDR3 encoded by the nucleotide sequence set forth in SEQ ID NO: 13 or sequences at least 80% identical thereto.

In further embodiments, the antibody or binding fragment thereof is characterized in comprising a heavy chain variable region comprising at least a CDR1 encoded by the nucleotide sequence set forth in SEQ ID NO: 15 or sequences at least 80% identical thereto, and/or a CDR2 encoded by the nucleotide sequence set forth in SEQ ID NO: 16 or sequences at least 80% identical thereto, and/or a CDR3 encoded by the nucleotide sequence set forth in SEQ ID NO: 17 or sequences at least 80% identical thereto.

In some embodiments, the antibody or binding fragment thereof is characterized in comprising a heavy chain variable region comprising at least a CDR1 encoded by the nucleotide sequence set forth in SEQ ID NO: 15 or sequences at least 80% identical thereto, a CDR2 encoded by the nucleotide sequence set forth in SEQ ID NO: 16 or sequences at least 80% identical thereto, and a CDR3 encoded by the nucleotide sequence set forth in SEQ ID NO: 17 or sequences at least 80% identical thereto.

In particular embodiments, the antibody or binding fragment thereof is characterized in that the light chain variable region comprises at least a CDR1 encoded by the nucleotide sequence set forth in SEQ ID NO: 11 or sequences at least 80% identical thereto, a CDR2 encoded by the nucleotide sequence set forth in SEQ ID NO: 12 or sequences at least 80% identical thereto, and/or a CDR3 encoded by the nucleotide sequence set forth in SEQ ID NO: 13 or sequences at least 80% identical thereto; and/or in that the heavy chain variable region comprises at least a CDR1 encoded by the nucleotide sequence set forth in SEQ ID NO: 15 or sequences at least 80% identical thereto, a CDR2 encoded by the nucleotide sequence set forth in SEQ ID NO: 16 or sequences at least 80% identical thereto, and/or a CDR3 encoded by the nucleotide sequence set forth in SEQ ID NO: 17 or sequences at least 80% identical thereto.

In some such embodiments, the antibody or binding fragment thereof is characterized in that the light chain variable region comprises at least a CDR1 encoded by the nucleotide sequence set forth in SEQ ID NO: 11 or sequences at least 80% identical thereto, a CDR2 encoded by the nucleotide sequence set forth in SEQ ID NO: 12 or sequences at least 80% identical thereto, and a CDR3 encoded by the nucleotide sequence set forth in SEQ ID NO: 13 or sequences at least 80% identical thereto; and/or in that the heavy chain variable region comprises at least a CDR1 encoded by the nucleotide sequence set forth in SEQ ID NO: 15 or sequences at least 80% identical thereto, a CDR2 encoded by the nucleotide sequence set forth in SEQ ID NO: 16 or sequences at least 80% identical thereto, and a CDR3 encoded by the nucleotide sequence set forth in SEQ ID NO17 or sequences at least 80% identical thereto.

In some embodiments, the antibody or binding fragment thereof is characterized in that the light chain variable region comprises at least a CDR1 encoded by the nucleotide sequence set forth in SEQ ID NO: 11 or sequences at least 80% identical thereto, a CDR2 encoded by the nucleotide sequence set forth in SEQ ID NO: 12 or sequences at least 80% identical thereto, and a CDR3 encoded by the nucleotide sequence set forth in SEQ ID NO: 13 or sequences at least 80% identical thereto; and in that the heavy chain variable region comprises at least a CDR1 encoded by the nucleotide sequence set forth in SEQ ID NO: 15 or sequences at least 80% identical thereto, a CDR2 encoded by the nucleotide sequence set forth in SEQ ID NO: 16 or sequences at least 80% identical thereto, and a CDR3 encoded by the nucleotide sequence set forth in SEQ ID NO: 17 or sequences at least 80% identical thereto.

In various embodiments, the antibody or binding fragment thereof is characterized in comprising a light chain comprising or consisting of an amino acid sequence encoded by the nucleotide sequence set forth in SEQ ID NO: 10 or sequences at least 80% identical thereto.

In various embodiments, the antibody or binding fragment thereof is characterized in comprising a heavy chain comprising or consisting of an amino acid sequence encoded by the nucleotide sequence set forth in SEQ ID NO: 14 or sequences at least 80% identical thereto.

In various embodiments, the antibody or binding fragment thereof is characterized in comprising a light chain comprising or consisting of an amino acid sequence encoded by the nucleotide sequence set forth in SEQ ID NO: 10 or sequences at least 80% identical thereto and a heavy chain comprising or consisting of an amino acid sequence encoded by the nucleotide sequence set forth in SEQ ID NO: 14 or sequences at least 80% identical thereto.

In various embodiments, the antibody or binding fragment thereof is characterized in comprising a light chain consisting of an amino acid sequence encoded by the nucleotide sequence set forth in SEQ ID NO: 10 or sequences at least 80% identical thereto and a heavy chain consisting of an amino acid sequence encoded by the nucleotide sequence set forth in SEQ ID NO: 14 or sequences at least 80% identical thereto.

**Table 1: MDG827 Antibody sequences overview.**

| **Antibody section** | **Amino acid sequence** | **Nucleic acid sequence** |
|---|---|---|
| Light chain (variable and constant regions) | SEQ ID NO: 2 | SEQ ID NO: 10 |
| CDR1 (CDR-L1) | SEQ ID NO: 3 | SEQ ID NO: 11 |
| CDR2 (CDR-L2) | SEQ ID NO: 4 | SEQ ID NO: 12 |
| CDR3 (CDR-L3) | SEQ ID NO: 5 | SEQ ID NO: 13 |
| Heavy chain (variable and constant regions) | SEQ ID NO: 6 | SEQ ID NO: 14 |
| CDR1 (CDR-H1) | SEQ ID NO: 7 | SEQ ID NO: 15 |
| CDR2 (CDR-H2) | SEQ ID NO: 8 | SEQ ID NO: 16 |
| CDR3 (CDR-H3) | SEQ ID NO: 9 | SEQ ID NO: 17 |

The structural characteristics of the antibody or binding fragment thereof according to the present invention, in some particular embodiments, allow for the tagging of a protein, preferably a TCR, comprising an amino acid sequence as defined herein, *e.g.,* an amino acid sequence having at least 80 %, such as 83 %, preferably at least 90 %, for instance 100 % sequence identity to the amino acid sequence set forth in SEQ ID NO: 62 (EVPKX₁R), such as further detailed in related embodiments, as outlined herein above. If it is stated that an antibody or fragment thereof binds to such a protein, preferably TCR, as defined herein, this means that the antibody or fragments thereof bind specifically to a portion of the protein, preferably TCR, that comprises or consists of the amino acid sequence having at least 80 %, such as 83 %, preferably at least 90 %, for instance 100 % sequence identity to the amino acid sequence set forth in SEQ ID NO: 62 (EVPKX₁R), such as further detailed in related embodiments, as outlined herein above.

For example, an antibody or fragment is specific for its cognate antigen when the variable regions of the antibody or fragment recognize and bind the cognate antigen with a detectable preference distinguishing the antigen from other known polypeptides of similar but not identical sequence by virtue of measurable differences in binding affinity. It will be understood that specific antibodies and fragments may also interact with other proteins (for example, *S. aureus* protein A or other antibodies in ELISA techniques) through interactions with sequences outside the variable region of the antibodies, and in particular, in the constant region of the antibody or fragment. Screening assays to determine binding specificity of an antibody are well known and routinely practiced in the art. For a comprehensive discussion of such assays (see e.g. 4. Harlow et al. (Eds), Antibodies A Laboratory Manual; Cold Spring Harbor Laboratory; Cold Spring Harbor, NY (1988), Chapter 6).

The antibodies and binding fragments thereof as they are used in the context of the present invention may be preferably monoclonal and more preferably monoclonal chimeric, murine, humanized or human antibodies. A particularly preferred aspect which applies to all embodiments described herein relates to monoclonal humanized antibodies or binding fragments thereof.

In various embodiments, the antibody of the present invention is a human IgG1-chimeric antibody and comprises a light chain comprising or consisting of an amino acid sequence having at least 80 %, preferably at least 85 %, more preferably at least 90 %, still more preferably at least 95 %, such as 100 % sequence identity to the amino acid sequence set forth in SEQ ID NO: 30, and further comprises a heavy chain comprising or consisting of an amino acid sequence having at least 80 %, preferably at least 85 %, more preferably at least 90 %, still more preferably at least 95 %, such as 100 % sequence identity to the amino acid sequence set forth in SEQ ID NO: 31.

In some embodiments, the antibody of the present invention is a human IgG1-chimeric antibody and comprises a light chain comprising or consisting of an amino acid sequence encoded by a nucleotide sequence having at least 80 %, preferably at least 85 %, more preferably at least 90 %, still more preferably at least 95 %, such as 100 % sequence identity to the nucleotide sequence set forth in SEQ ID NO: 32, and further comprises a heavy chain comprising or consisting of an amino acid sequence encoded by a nucleotide sequence having at least 80 %, preferably at least 85 %, more preferably at least 90 %, still more preferably at least 95 %, such as 100 % sequence identity to the nucleotide sequence set forth in SEQ ID NO: 33.

In other embodiments, the antibody of the present invention is a humanized antibody and comprises a light chain comprising or consisting of an amino acid sequence having at least 80 %, preferably at least 85 %, more preferably at least 90 %, still more preferably at least 95 %, such as 100 % sequence identity to the amino acid sequence set forth in SEQ ID NO: 34.

In some embodiments, the antibody of the present invention is a humanized antibody and comprises a light chain comprising or consisting of an amino acid sequence encoded by a nucleotide sequence having at least 80 %, preferably at least 85 %, more preferably at least 90 %, still more preferably at least 95 %, such as 100 % sequence identity to the nucleotide sequence set forth in SEQ ID NO: 36.

In various embodiments, the antibody of the present invention is a humanized antibody and comprises a heavy chain comprising or consisting of an amino acid sequence having at least 80 %, preferably at least 85 %, more preferably at least 90 %, still more preferably at least 95 %, such as 100 % sequence identity to the amino acid sequence set forth in SEQ ID NO: 35.

In some embodiments, the antibody of the present invention is a humanized antibody and comprises a heavy chain comprising or consisting of an amino acid sequence encoded by a nucleotide sequence having at least 80 %, preferably at least 85 %, more preferably at least 90 %, still more preferably at least 95 %, such as 100 % sequence identity to the nucleotide sequence set forth in SEQ ID NO: 37.

In some embodiments, the antibody of the present invention is a humanized antibody and comprises a light chain comprising or consisting of an amino acid sequence having at least 80 %, preferably at least 85 %, more preferably at least 90 %, still more preferably at least 95 %, such as 100 % sequence identity to the amino acid sequence set forth in SEQ ID NO: 34 and further comprises a heavy chain comprising or consisting of an amino acid sequence having at least 80 %, preferably at least 85 %, more preferably at least 90 %, still more preferably at least 95 %, such as 100 % sequence identity to the amino acid sequence set forth in SEQ ID NO: 35.

In some embodiments, the antibody of the present invention is a humanized antibody and comprises a light chain consisting of an amino acid sequence having at least 80 %, preferably at least 85 %, more preferably at least 90 %, still more preferably at least 95 %, such as 100 % sequence identity to the amino acid sequence set forth in SEQ ID NO: 34 and further comprises a heavy chain consisting of an amino acid sequence having at least 80 %, preferably at least 85 %, more preferably at least 90 %, still more preferably at least 95 %, such as 100 % sequence identity to the amino acid sequence set forth in SEQ ID NO: 35.

The antibodies can be of different subtypes, such as of the IgG or IgM class. Antibodies of the IgG class are of particular interest. In particular embodiments, the antibody or binding fragment thereof belongs to the lgG2a subtype, preferably the rat IgG2a subtype, particularly comprising a kappa light chain.

Antibodies or binding fragments as described herein are preferably capable of tagging a protein, preferably a T cell receptor, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), particular embodiments of which are defined herein above.

These properties of the antibodies or binding fragments described herein may thus allow target-specific binding of proteins, preferably TCRs, comprising the amino acid sequence set forth in SEQ ID NO: 62 (EVPKX₁R), as herein defined and described, a property which is particularly useful for protein, e.g., TCR detection and/or visualization purposes, which may be applied both in research and therapeutic scenarios.

Preferably, in all the aforementioned embodiments, the sequence identity is at least about 85%, more preferably at least about 90%, even more preferably at least about 95% and most preferably at least about 98% or about 99%. Sequence identity may be determined over the whole length of the respective sequences.

The determination of percent identity between two sequences is preferably accomplished using the mathematical algorithm of Karlin and Altschul (1993) Proc. Natl. Acad. Sci USA 90: 5873-5877. Such an algorithm is incorporated into the BLASTn and BLASTp programs of Altschul et al. (1990) J. Mol. Biol,. 215: 403-410 (see references) available at NCBI (http://www.ncbi.nlm.nih.gov/blast/Blast.cge).

The determination of percent identity is performed with the standard parameters of the BLASTn and BLASTp programs.

BLAST polynucleotide searches are performed with the BLASTn program.

For the general parameters, the "Max Target Sequences" box may be set to 100, the "Short queries" box may be ticked, the "Expect threshold" box may be set to 10 and the "Word Size" box may be set to 28. For the scoring parameters the "Match/mismatch Scores" may be set to 1, -2 and the "Gap Costs" box may be set to linear. For the Filters and Masking parameters, the "Low complexity regions" box may not be ticked, the "Species-specific repeats" box may not be ticked, the "Mask for lookup table only" box may be ticked, the "Mask lower case letters" box may not be ticked.

BLAST protein searches are performed with the BLASTp program. For the general parameters, the "Max Target Sequences" box may be set to 100, the "Short queries" box may be ticked, the "Expect threshold" box may be set to 10 and the "Word Size" box may be set to "3". For the scoring parameters the "Matrix" box may be set to "BLOSUM62", the "Gap Costs" Box may be set to "Existence: 11 Extension:1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked.

The term "CDR" refers to the complementarity determining region or hypervariable region amino acid residues of an antibody that participate in or are responsible for antigen-binding. The CDRs as described herein are defined according to the international ImMunoGeneTics information system^{®} (LaFranc, et al. 2005. Nucl Acids Res. 33:D593-D597) and as described in (Lefranc et al. Dev. Comparat. Immunol. 27:55-77, 2003).

The above-mentioned CDRs of a light and heavy chain variable region may be embedded in human sequences of framework and constant regions derived from other human antibodies, particularly if such sequences have been shown to be effective in antibody dependent cell mediated cytotoxicity (ADCC). In this context, one may, e.g., use the human constant and framework sequences of humanized therapeutic antibodies that have been successfully used for therapeutic applications. The above-mentioned CDRs of a light and heavy chain variable region are preferably incorporated into the framework and constant regions of such humanized antibodies of the human IgG class.

Further, the above-mentioned CDRs of a light and heavy chain variable region may be embedded in essentially human sequences for framework and constant regions. However, particularly the framework regions, but also the constant regions may comprise amino acids as they are, e.g., typically found in mouse antibodies which are known to enhance antigen binding and/or e.g. ADCC (see, e.g., European patent application EP 0 451 216). Preferably these antibodies are of the IgG class.

In the following several methodologies are described, which have been developed for reduction of immunogenicity of non-human derived antibodies, like chimerization or humanization generally known in the art.

During humanization, all amino acids, which are not essential for proper antibody folding or antigen recognition, are exchanged with amino acids from the human antibody counterpart. Several methods for mab humanization are developed including traditional CDR grafting or more novel approaches which involve computer modeling and bioinformatics analysis. Humanization of the heavy and light chains of CL 1 was performed using the CDR grafting method (see e.g. Desmet et al. in Kontermann and Dübel (eds.) Antibody Engineering Vol. 1, p. 341ff; Bernett et al. J. Mol. Biol. (2010) 396, 1474-1490). The heavy and light chain variable framework regions can be derived from the same or different human antibody sequences. The human antibody sequences can be the sequences of naturally occurring human antibodies. Human heavy and light chain variable framework regions are listed, e.g., in Lefranc, M.-P., Current Protocols in Immunology (2000) - Appendix IP A.1P.1-A.1P.37 and are accessible via IMGT, the international ImMunoGeneTics information system^{®} (http://imgt.cines.fr).

Epitope mapping may be undertaken by producing different fragments of the antigen, as herein defined, and to then test these fragments for binding to antibodies or the binding fragments thereof. Binding may be measured using a Biacore^{®} interaction analysis. One may also use commercially available peptide arrays such as PepSpot^{™} from JPT Peptide Technologies GmbH (Berlin, Germany), or proteomics-based mass spectrometry methods. Competition for binding to a particular antigen or epitope can be determined using assays known in the art. For example, one may label an antibody in accordance with the invention and test for its binding to the amino acid sequence set forth in SEQ ID NO: 62 (EVPKX₁R), for instance. Subsequently, one adds an unlabeled equivalent antibody and determines whether it affects binding of the labeled antibody, or binding of the labeled antibody is studied in presence or absence of various concentrations of such unlabeled antibodies. Such label could be radioactive or fluorescent or other kinds of detectable label.

Competition for binding to a particular antigen or epitope is determined by a reduction in binding to antigen or epitope of at least about 50%, or at least about 70%, or at least about 80%, or at least about 90%, or at least about 95%, or at least about 99% or about 100% for the antibody in accordance with the invention. Binding may be measured using Biacore^{®} equipment, various fluorescence detection technologies (e.g., fluorescence correlation spectroscopy, fluorescence cross-correlation, fluorescence lifetime measurements etc.) or various types of radioimmunoassays or other assays used to follow antibody binding to a target molecule.

The present invention further considers cluster-specific antibodies or binding fragments thereof. A full-length antibody includes a constant domain and a variable domain. The constant region need not be present in an antigen binding fragment of an antibody.

Binding fragments may thus include portions of an intact full-length antibody, such as an antigen binding or variable region of the complete antibody. Examples of antibody fragments include Fab, F(ab')₂, Id and Fv fragments; diabodies; linear antibodies; single-chain antibody molecules (e.g., scFv); multispecific antibody fragments such as bispecific, trispecific, and multispecific antibodies (e.g., diabodies, triabodies, tetrabodies); minibodies; chelating recombinant antibodies; tribodies or bibodies; intrabodies; nanobodies; small modular immunopharmaceuticals (SMIP), binding-domain immunoglobulin fusion proteins; camelized antibodies; VHH containing antibodies; chimeric antigen receptor (CAR); and any other polypeptides formed from antibody fragments. The skilled person is aware that the antigen-binding function of an antibody can be performed by fragments of a full-length antibody.

A Fab fragment consists of the VL, VH, CL and CH1 domains. An F(ab')² fragment comprises two Fab fragments linked by a disulfide bridge at the hinge region. An Fd is the VH and CH1 domains of a single arm of an antibody. An Fv fragment is the VL and VH domains of a single arm of an antibody.

Binding fragments also encompass monovalent or multivalent, or monomeric or multimeric (e.g., tetrameric), CDR-derived binding domains.

A bispecific antibody comprises two different binding specificities and thus binds to two different antigens. In one embodiment, the bispecific antibody comprises a first antigen recognition domain that binds to a first antigen and a second antigen recognition domain that binds to a second antigen. In one embodiment, the first antigen recognition domain binds to an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R) and the second antigen recognition region binds to an epitope that is different from the epitope that is recognized by the first antigen recognition domain, as defined herein.

Methods for making bispecific antibodies are known to the skilled person in the art. Bispecific antibodies can be produced recombinantly using the co-expression of two immunoglobulin heavy chain/light chain pairs, as for example described in Milstein et al. (1983; Nature 305:537). Alternatively, bispecific antibodies can be prepared using chemical linkage (see, *e.g.,* Brennan *et al.* (1985)). Bispecific antibodies include bispecific antibody fragments (see, *e.g.,* Holliger et al. (1993) Proc. Natl. Acad. Sci. U.S.A. 90:6444-48, Gruber et al. (1994) J. Immunol. 152:5368.)

A chimeric antigen receptor CAR comprises an antigen binding domain derived from a bispecific antibody, a transmembrane domain, and a CD3 zeta signaling domain.

More specifically the term "chimeric antigen receptors (CARs)," as used herein, refers for example to chimeric T-cell receptors, artificial T-cell receptors, or chimeric immunoreceptors. CARs may be used for mediating the specificity of a monoclonal antibody onto a T cell. In particular aspects, CARs comprise fusions of single-chain variable fragments (scFv) derived from monoclonal antibodies, fused to CD3-zeta a transmembrane domain and endodomain. In certain cases, the spacing of the antigen-recognition domain can be modified to reduce activation-induced cell death. In certain cases, CARs comprise domains for additional co-stimulatory signaling, such as CD3-zeta, FcR, CD27, CD28, CD137, DAP10, and/or OX40.

The antibodies and binding fragments thereof of the present invention may also encompass variants of the exemplary antibodies, binding fragments and sequences disclosed herein.

Variants include peptides and polypeptides comprising one or more amino acid sequence substitutions, deletions, and/or additions that have the same or substantially the same affinity and specificity of epitope binding as one or more of the exemplary antibodies, fragments and sequences disclosed herein. Thus, variants include peptides and polypeptides comprising one or more amino acid sequence substitutions, deletions, and/or additions to the exemplary antibodies, fragments and sequences disclosed herein where such substitutions, deletions and/or additions do not cause substantial changes in affinity and specificity of epitope binding. For example, a variant of an antibody or fragment may result from one or more changes to an antibody or fragment comprising one or more of amino acid sequence of SEQ ID NOs: 2 and 6 or where the changed antibody or fragment has the same or substantially the same affinity and specificity of epitope binding as the starting sequence.

Antibodies or binding fragments thereof as far as they are generally referred to in the context of the present invention may also be part of larger immunoadhesion molecules, formed by covalent or non-covalent association of the antibody or antibody portion with e.g. one or more other proteins or peptides. Examples of such immunoadhesion molecules include use of the streptavidin core region to make a tetrameric scFv molecule (Kipriyanov, S. M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S. M., et al. (1994) Mol. Immunol. 31:1047-1058). Antibodies and fragments comprising immunoadhesion molecules can be obtained using standard recombinant DNA techniques, as described herein. Preferred antigen binding portions are complete domains or pairs of complete domains.

The antibodies and binding fragments of the present invention may also encompass domain antibody (dAb) fragments (Ward et al., Nature 341:544-546, 1989) which consist of a V_{H} domain. The antibodies and binding fragments of the present invention also encompass diabodies are bivalent antibodies in which V_{H} and V_{L} domains are expressed on a single polypeptide chain, but using a linker that is too short to allow for pairing between the two domains on the same chain, thereby forcing the domains to pair with complementary domains of another chain and creating two antigen binding sites (see e.g., EP 404,097; WO 93/11161; Holliger et al., Proc. Natl. Acad. Sci. USA 90:6444-6448, 1993, and Poljak et al., Structure 2:1121-1123, 1994). Diabodies can be bispecific or monospecific.

As mentioned, the antibodies and binding fragments of the present invention also encompass single-chain antibody fragments (scFv). An scFv comprises an antibody heavy chain variable region (V_{H}) operably linked to an antibody light chain variable region (V_{L}) wherein the heavy chain variable region and the light chain variable region, together or individually, form a binding site. A scFv may comprise a V_{H} region at the amino-terminal end and a V_{L} region at the carboxy-terminal end. Alternatively, scFv may comprise a V_{L} region at the amino-terminal end and a V_{H} region at the carboxy-terminal end. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883).

A scFv may optionally further comprise a polypeptide linker between the heavy chain variable region and the light chain variable region. Such polypeptide linkers generally comprise between 1 and 50 amino acids, alternatively between 3 and 12 amino acids, alternatively 2 amino acids. An example of a linker peptide for linking heavy and light chains in a scFv comprises the 5 amino acid sequence Gly-Gly-Gly-Gly-Ser (SEQ ID NO: 90). Other examples comprise one or more tandem repeats of this sequence (for example, a polypeptide comprising two to four repeats of Gly-Gly-Gly-Gly-Ser (SEQ ID NO: 90) to create linkers.

The antibodies and binding fragments of the present invention may also encompass heavy chain antibodies (HCAb). Exceptions to the H₂L₂ structure of conventional antibodies occur in some isotypes of the immunoglobulins found in camelids (camels, dromedaries and llamas; Hamers-Casterman et al., 1993 Nature 363: 446; Nguyen etal., 1998 J. Mol. Biol. 275: 413), wobbegong sharks (Nuttall et al., Mol Immunol. 38:313-26, 2001), nurse sharks (Greenberg et al., Nature 374:168-73, 1995; Roux et al., 1998 Proc. Nat. Acad. Sci. USA 95: 11804), and in the spotted ratfish (Nguyen, etal., "Heavy-chain antibodies in Camelidae; a case of evolutionary innovation," 2002 Immunogenetics 54(1): 39-47). These antibodies can apparently form antigen-binding regions using only heavy chain variable region, in that these functional antibodies are dimers of heavy chains only (referred to as "heavy-chain antibodies" or "HCAbs"). Accordingly, some embodiments of the present antibodies and binding fragments may be heavy chain antibodies (HCAb) that specifically bind to the TCR. For example, heavy chain antibodies that are a class of IgG and devoid of light chains are produced by animals of the genus *Camelidae* which includes camels, dromedaries and llamas (Hamers-Casterman et al., Nature 363:446-448 (1993)). HCAbs have a molecular weight of about 95 kDa instead of the about 160 kDa molecular weight of conventional IgG antibodies. Their binding domains consist only of the heavy-chain variable domains, often referred to as V_{HH} to distinguish them from conventional V_{H}, Muyldermans et al., J. Mol. Recognit. 12:131-140 (1999). The variable domain of the heavy-chain antibodies is sometimes referred to as a nanobody (Cortez-Retamozo et al., Cancer Research 64:2853-57, 2004). A nanobody library may be generated from an immunized dromedary as described in Conrath et al., (Antimicrob Agents Chemother 45: 2807-12, 2001) or using recombinant methods.

Since the first constant domain (C_{H1}) is absent (spliced out during mRNA processing due to loss of a splice consensus signal), the variable domain (V_{HH}) is immediately followed by the hinge region, the C_{H2} and the C_{H3} domains (Nguyen et al., Mol. Immunol. 36:515-524 (1999); Woolven et al., Immunogenetics 50:98-101 (1999)). Camelid V_{HH} reportedly recombines with IgG2 and IgG3 constant regions that contain hinge, C_{H2}, and C_{H3} domains and lack a C_{H}, domain (Hamers-Casterman *et al., supra*). For example, llama IgG1 is a conventional (H₂L₂) antibody isotype in which V_{H} recombines with a constant region that contains hinge, C_{H1}, C_{H2} and C_{H3} domains, whereas the llama IgG2 and IgG3 are heavy chain-only isotypes that lack C_{H1} domains and that contain no light chains.

Although the HCAbs are devoid of light chains, they have an antigen-binding repertoire. The genetic generation mechanism of HCAbs is reviewed in Nguyen et al. Adv. Immunol 79:261-296 (2001) and Nguyen etal., Immunogenetics 54:39-47 (2002). Sharks, including the nurse shark, display similar antigen receptor-containing single monomeric V-domains. Irving et al., J. Immunol. Methods 248:31-45 (2001); Roux et al., Proc. Natl. Acad. Sci. USA 95:11804 (1998).

V_{HH}s comprise small intact antigen-binding fragments (for example, fragments that are about 15 kDa, 118-136 residues). Camelid V_{HH} domains have been found to bind to antigen with high affinity (Desmyter et al., J. Biol. Chem. 276:26285-90, 2001), with V_{HH} affinities typically in the nanomolar range and comparable with those of Fab and scFv fragments. V_{HH}s are highly soluble and more stable than the corresponding derivatives of scFv and Fab fragments. V_{H} fragments have been relatively difficult to produce in soluble form, but improvements in solubility and specific binding can be obtained when framework residues are altered to be more V_{HH}-like (see, for example, Reichman et al., J Immunol Methods 1999, 231:25-38). V_{HH}s carry amino acid substitutions that make them more hydrophilic and prevent prolonged interaction with BiP (Immunoglobulin heavy-chain binding protein), which normally binds to the H-chain in the Endoplasmic Reticulum (ER) during folding and assembly, until it is displaced by the L-chain. Because of the V_{HH}s' increased hydrophilicity, secretion from the ER is improved.

Functional V_{HH}s may be obtained by proteolytic cleavage of HCAb of an immunized camelid, by direct cloning of V_{HH} genes from B-cells of an immunized camelid resulting in recombinant V_{HH}s, or from naive or synthetic libraries. V_{HH}s with desired antigen specificity may also be obtained through phage display methodology. Using V_{HH}s in phage display is much simpler and more efficient compared to Fabs or scFvs, since only one domain needs to be cloned and expressed to obtain a functional antigen-binding fragment. Muyldermans, Biotechnol. 74:277-302 (2001); Ghahroudi etal., FEBS Lett. 414:521-526 (1997); and van der Linden et al., J. Biotechnol. 80:261-270 (2000). Methods for generating antibodies having camelid heavy chains are also described in U.S. Patent Publication Nos. 20050136049 and 20050037421.

The binding antibodies and binding fragments thereof may also encompass any of the, e.g., foregoing specifically mentioned amino acid sequences of the light or heavy chains with one or more conservative substitutions (*e.g.,* 1, 2, 3, 4, 5, 6, 7, 8, 9, 10, 11, 12, 13, 14, or 15 conservative substitutions). One can determine the positions of an amino acid sequence that are candidates for conservative substitutions, and one can select synthetic and naturally-occurring amino acids that effect conservative substitutions for any particular amino acids. Consideration for selecting conservative substitutions include the context in which any particular amino acid substitution is made, the hydrophobicity or polarity of the side-chain, the general size of the side chain, and the pK value of side-chains with acidic or basic character under physiological conditions. For example, lysine, arginine, and histidine are often suitably substituted for each other. As is known in the art, this is because all three amino acids have basic side chains, whereas the pK value for the side-chains of lysine and arginine are much closer to each other (about 10 and 12) than to histidine (about 6). Similarly, glycine, alanine, valine, leucine, and isoleucine are often suitably substituted for each other, with the proviso that glycine is frequently not suitably substituted for the other members of the group. Other groups of amino acids frequently suitably substituted for each other include, but are not limited to, the group consisting of glutamic and aspartic acids; the group consisting of phenylalanine, tyrosine, and tryptophan; and the group consisting of serine, threonine, and, optionally, tyrosine.

By making conservative modifications to the amino acid sequence or corresponding modifications to the encoding nucleotides, one can produce antibodies or binding fragments thereof having functional and chemical characteristics similar to those of the exemplary antibodies and fragments disclosed herein.

The binding antibodies and binding fragments thereof as they are mentioned in the context of the present invention may encompass derivatives of the exemplary antibodies, fragments and sequences disclosed herein. Derivatives include polypeptides or peptides, or variants, fragments or derivatives thereof, which have been chemically modified. Examples include covalent attachment of one or more polymers, such as water-soluble polymers, N-linked, or O-linked carbohydrates, sugars, phosphates, and/or other such molecules such as detectable labels such as fluorophores.

Labeling agents may be coupled either directly or indirectly to the antibodies or antigens of the invention. One example of indirect coupling is by use of a spacer moiety. Furthermore, the antibodies of the present invention can comprise a further domain, said domain being linked by covalent or noncovalent bonds. The linkage can be based on genetic fusion according to the methods known in the art and described above or can be performed by, *e.g.,* chemical cross-linking as described in, *e.g.,* international application WO 94/04686. The additional domain present in the fusion protein comprising the antibody of the invention may preferably be linked by a flexible linker, advantageously a polypeptide linker, wherein said polypeptide linker comprises plural, hydrophilic, peptide-bonded amino acids of a length sufficient to span the distance between the C-terminal end of said further domain and the N-terminal end of the antibody of the invention or vice versa. The therapeutically or diagnostically active agent can be coupled to the antibody of the invention or an antigen-binding fragment thereof by various means. This includes, for example, single-chain fusion proteins comprising the variable regions of the antibody of the invention coupled by covalent methods, such as peptide linkages, to the therapeutically or diagnostically active agent. Further examples include molecules which comprise at least an antigen-binding fragment coupled to additional molecules covalently or non-covalently include those in the following non-limiting illustrative list. Traunecker et al., Int. J. Cancer Surp. SuDP 7 (1992), 51-52, describe the bispecific reagent janusin in which the Fv region directed to CD3 is coupled to soluble CD4 or to other ligands such as OVCA and IL-7. Similarly an Fv region directed to TCR Vα chains or TCR Vβ chains may be coupled to portions of e.g. an anti-CD40 agonistic antibody and/or portions of an anti-CTLA4 antagonistic antibody. Similarly, the variable regions of the antibody of the invention can be constructed into Fv molecules and coupled to alternative ligands such as those illustrated in the cited article. Higgins et al., J. Infect Disease 166 (1992), 198-202, described a hetero-conjugated antibody composed of OKT3 cross-linked to an antibody directed to a specific sequence in the V3 region of GP120. Such hetero-conjugate antibodies can also be constructed using at least the variable regions contained in the antibody of the invention methods. Additional examples of specific antibodies include those described by Fanger et al., Cancer Treat. Res. 68 (1993), 181-194 and by Fanger et al., Crit. Rev. Immunol. 12 (1992), 101-124. Conjugates that are immunotoxins including conventional antibodies have been widely described in the art. The toxins may be coupled to the antibodies by conventional coupling techniques or immunotoxins containing protein toxin portions can be produced as fusion proteins. The antibodies of the present invention can be used in a corresponding way to obtain such immunotoxins. Illustrative of such immunotoxins are those described by Byers et al., Seminars Cell. Biol. 2 (1991), 59-70 and by Fanger et al., Immunol. Today 12 (1991), 51-54.

The above-described fusion proteins may further comprise a cleavable linker or cleavage site for proteases. These spacer moieties, in turn, can be either insoluble or soluble (Diener et al., Science 231 (1986), 148) and can be selected to enable drug release from the antigen at the target site.

Examples of therapeutic agents, which can be coupled to the antibodies and antigens of the present invention for immunotherapy, are drugs, radioisotopes, lectins, and toxins. The drugs with which can be conjugated to the antibodies and antigens of the present invention include compounds which are classically referred to as drugs such as mitomycin C, daunorubicin, and vinblastine. In using radioisotopically conjugated antibodies or antigens of the invention for, *e.g.,* tumor immunotherapy, certain isotopes may be more preferable than others depending on such factors as leukocyte distribution as well as stability and emission.

Some emitters may be preferable to others. In general, alpha and beta particle emitting radioisotopes are preferred in immunotherapy. Preferred are short range high energy a emitters such as ²¹²Bi. Examples of radioisotopes which can be bound to the antibodies or antigens of the invention for therapeutic purposes are ¹²⁵I, ¹³¹I, ⁹⁰Y, ⁶⁷Cu, ²¹²Bi, ²¹²At, ²¹¹Pb, ⁴⁷Sc, ¹⁰⁹Pd and ¹⁸⁸Re. Other therapeutic agents which can be coupled to the antibody or antigen of the invention, as well as ex *vivo* and *in vivo* therapeutic protocols, are known, or can be easily ascertained, by those of ordinary skill in the art.

The invention also relates, in some embodiment, to nucleic acid molecules encoding antibodies and binding fragments thereof, vectors comprising such nucleic acid molecules and host cells comprising such nucleic acid sequences and vectors.

The antibodies and binding fragments thereof may be encoded by a single nucleic acid (*e.g.,* a single nucleic acid comprising nucleotide sequences that encode the light and heavy chain polypeptides of the antibody), or by two or more separate nucleic acids, each of which encode a different part of the antibody or antibody fragment. In this regard, the invention provides one or more nucleic acids that encode any of the forgoing antibodies, or binding fragments. The nucleic acid molecules may be DNA, cDNA, RNA and the like.

According to one aspect of the invention, the invention provides a nucleic acid that encodes a heavy chain region of an antibody or a portion thereof. An exemplary nucleic acid sequence is provided in SEQ ID NO: 14. The invention also provides a nucleic acid that encodes a light chain variable region of an antibody or a portion thereof. An exemplary nucleic acid sequence is provided in SEQ ID NO:10.

Also encompassed by the invention are nucleic acids encoding any of the foregoing amino acid sequences of the light or heavy chains that comprise one or more conservative substitutions (*e.g.,* 1, 2, 3, 4, 5, 6, 7, 8, 9, 10, 11, 12, 13, 14, or 15 conservative substitutions), as discussed with respect to the antibody and antibody fragment of the invention, where the antibody or fragment comprising the substitution has the same or substantially the same affinity and specificity of epitope binding as one or more of the exemplary antibodies, fragments and sequences disclosed herein.

Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally associated or heterologous promoter regions.

The nucleic acids described herein can be inserted into vectors, *e.g.,* nucleic acid expression vectors and/or targeting vectors. Such vectors can be used in various ways, *e.g.,* for the expression of an antibody or a binding fragment in a cell or transgenic animal. Accordingly, the invention provides a vector comprising any one or more of the nucleic acids of the invention. A "vector" is any molecule or composition that has the ability to carry a nucleic acid sequence into a suitable host cell where synthesis of the encoded polypeptide can take place. Typically and preferably, a vector is a nucleic acid that has been engineered, using recombinant DNA techniques that are known in the art, to incorporate a desired nucleic acid sequence (e.g., a nucleic acid of the invention). Desirably, the vector is comprised of DNA. However, vectors that are not based on nucleic acids, such as liposomes, are also known in the art and can be used in connection with the invention. The inventive vector can be based on a single type of nucleic acid (*e.g.,* a plasmid) or non-nucleic acid molecule (*e.g.,* a lipid or a polymer). Alternatively, the vector can be a combination of a nucleic acid and a non-nucleic acid (*i.e.,* a "chimeric" vector). For example, a plasmid harboring the nucleic acid can be formulated with a lipid or a polymer as a delivery vehicle. Such a vector is referred to herein as a "plasmid-lipid complex" and a "plasmid-polymer" complex, respectively. The inventive gene transfer vector can be integrated into the host cell genome or can be present in the host cell in the form of an episome.

Vectors are typically selected to be functional in the host cell in which the vector will be used (the vector is compatible with the host cell machinery such that amplification of the gene and/or expression of the gene can occur). A nucleic acid molecule encoding an antibody or binding fragment thereof may be amplified/expressed in prokaryotic, yeast, insect (baculovirus systems) and/or eukaryotic host cells.

Selection of the host cell will depend in part on whether the antibody or fragment is to be post-transitionally modified (*e.g.,* glycosylated and/or phosphorylated). If so, yeast, insect, or mammalian host cells are preferable.

Expression vectors typically contain one or more of the following components (if they are not already provided by the nucleic acid molecules): a promoter, one or more enhancer sequences, an origin of replication, a transcriptional termination sequence, a complete intron sequence containing a donor and acceptor splice site, a leader sequence for secretion, a ribosome binding site, a polyadenylation sequence, a polylinker region for inserting the nucleic acid encoding the polypeptide to be expressed, and a selectable marker element.

The invention in some aspects further provides a cell (*e.g.,* an isolated or purified cell) comprising a nucleic acid or vector of the invention. The cell can be any type of cell capable of being transformed with the nucleic acid or vector of the invention so as to produce a polypeptide encoded thereby. The cell is preferably the cell of a mammal, such as a human, and is more preferably a hybridoma cell, an embryonic stem cell, or a fertilized egg. The embryonic stem cell or fertilized egg may not be a human embryonic stem cell or a human fertilized egg.

The host cells may be prokaryotic host cells (such as *E. coli*) or eukaryotic host cells (such as a yeast cell, an insect cell, or a vertebrate cell). The host cell, when cultured under appropriate conditions, expresses an antibody or binding fragment which can subsequently be collected from the culture medium (if the host cell secretes it into the medium) or directly from the host cell producing it (if it is not secreted). Selection of an appropriate host cell will depend upon various factors, such as desired expression levels, polypeptide modifications that are desirable or necessary for activity, such as glycosylation or phosphorylation, and ease of folding into a biologically active molecule. A number of suitable host cells are known in the art and many are available from the American Type Culture Collection (ATCC), Manassas, Va. Examples include mammalian cells, such as Chinese hamster ovary cells (CHO) (ATCC No. CCL61) CHO DHFR-cells (Urlaub et al. Proc. Natl. Acad. Sci. USA 97, 4216-4220 (1980)), human embryonic kidney (HEK) 293 or 293T cells (ATCC No. CRL1573), 3T3 cells (ATCC No. CCL92), or PER.C6 cells.

The cell comprising the nucleic acid or vector of the invention can be used to produce the antibody or binding fragment thereof, or a portion thereof (*e.g.,* a heavy chain sequence, or a light chain sequence encoded by the nucleic acid or vector). After introducing the nucleic acid or vector of the invention into the cell, the cell is cultured under conditions suitable for expression of the encoded sequence. The antibody, antigen binding fragment, or portion of the antibody then can be isolated from the cell.

Another aspect of the invention relates to the use of an antibody or binding fragment thereof according to the present invention, *i.e.,* as herein defined and described, for tagging a protein, preferably a TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R).

Another aspect of the invention relates to the use of an antibody or binding fragment thereof according to the present invention, *i.e.,* as herein defined and described, for ex *vivo* tagging a protein, preferably a TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R).

More specific embodiments pertaining to said antigen have been outlined herein above in the context of the antibody or binding fragment thereof and apply, *mutatis mutandis,* likewise to this aspect of the present invention.

A further aspect of the invention relates to an antibody or binding fragment thereof as described herein for use as a medicament and/or for use as a screening agent.

A specific embodiment relates to an antibody or binding fragment according to the present invention for use in the therapy of malignancies, preferably for use in the treatment of cancer, viral diseases, or autoimmunity.

In one embodiment the cancer is a hematological cancer or a solid tumor.

Hematological cancers also called blood cancers which do not form solid tumors and therefore are primarily dispersed in the body. Examples of hematological cancers are leukemia, lymphoma or multiple myeloma.

The binding data showing that MDG827 binds to Jurkat cells, which are T cell leukemia cells and hence are an established model for T-cell leukemia, show that the antibody or binding fragments according to the invention are targeting T cell leukemia cells. Therefore, the antibodies or binding fragments of the invention can be used for the treatment of T cell mediated diseases such as T cell leukemia, lymphoma or multiple myeloma.

The antibodies or binding fragments thereof according to the present invention can be formulated in compositions, especially pharmaceutical compositions. Therefore, in a further aspect, the present invention also relates to a pharmaceutical composition comprising at least one antibody or binding fragment thereof in accordance with the present invention. Typically, such compositions comprise a therapeutically or prophylactically effective amount of an antibody or binding fragment thereof according to the present invention in admixture with one or more suitable agents, *e.g.,* pharmaceutically acceptable agents.

Pharmaceutically acceptable agents for use in the present pharmaceutical compositions include carriers, excipients, diluents, antioxidants, preservatives, coloring, flavoring and diluting agents, emulsifying agents, suspending agents, solvents, fillers, bulking agents, buffers, delivery vehicles, tonicity agents, cosolvents, wetting agents, complexing agents, buffering agents, antimicrobials, and surfactants.

The composition can be in liquid form or in a lyophilized or freeze-dried form and may include one or more lyoprotectants, excipients, surfactants, high molecular weight structural additives and/or bulking agents (see for example US Patents 6,685,940, 6,566,329, and 6,372,716).

Compositions can be suitable for parenteral administration. Exemplary compositions are suitable for injection or infusion into an animal by any route available to the skilled worker, such as intraarticular, subcutaneous, intravenous, intramuscular, intraperitoneal, intracerebral (intraparenchymal), intracerebroventricular, intramuscular, intraocular, intraarterial, or intralesional routes. A parenteral formulation typically will be a sterile, pyrogen-free, isotonic aqueous solution, optionally containing pharmaceutically acceptable preservatives.

Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringers' dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present, such as, for example, anti-microbials, anti-oxidants, chelating agents, inert gases and the like. See generally, Remington's Pharmaceutical Science, 16th Ed., Mack Eds., 1980.

Pharmaceutical compositions described herein can be formulated for controlled or sustained delivery in a manner that provides local concentration of the product (e.g., bolus, depot effect) and/or increased stability or half-life in a particular local environment. The compositions can include the formulation of antibodies, binding fragments, nucleic acids, or vectors of the invention with particulate preparations of polymeric compounds such as polylactic acid, polyglycolic acid, etc., as well as agents such as a biodegradable matrix, injectable microspheres, microcapsular particles, microcapsules, bioerodible particles beads, liposomes, and implantable delivery devices that provide for the controlled or sustained release of the active agent which then can be delivered as a depot injection.

Both biodegradable and non-biodegradable polymeric matrices can be used to deliver compositions of the present invention, and such polymeric matrices may comprise natural or synthetic polymers. Biodegradable matrices are preferred. The period of time over which release occurs is based on selection of the polymer. Typically, release over a period ranging from between a few hours and three to twelve months is most desirable.

Alternatively or additionally, the compositions can be administered locally *via* implantation into the affected area of a membrane, sponge, or other appropriate material on to which an antibody, binding fragment, nucleic acid, or vector of the invention has been absorbed or encapsulated. Where an implantation device is used, the device can be implanted into any suitable tissue or organ, and delivery of an antibody, binding fragment, nucleic acid, or vector of the invention can be directly through the device *via* bolus, or *via* continuous administration, or *via* catheter using continuous infusion.

A pharmaceutical composition comprising an antibody or binding fragment thereof according to the present invention can be formulated for inhalation, such as for example, as a dry powder. Inhalation solutions also can be formulated in a liquefied propellant for aerosol delivery. In yet another formulation, solutions may be nebulized.

Certain formulations containing antibodies or binding fragments thereof according to the present invention can be administered orally. Formulations administered in this fashion can be formulated with or without those carriers customarily used in the compounding of solid dosage forms such as tablets and capsules. For example, a capsule can be designed to release the active portion of the formulation at the point in the gastrointestinal tract when bioavailability is maximized and pre-systemic degradation is minimized. Additional agents can be included to facilitate absorption of a selective binding agent. Diluents, flavorings, low melting point waxes, vegetable oils, lubricants, suspending agents, tablet disintegrating agents, and binders also can be employed.

In a further aspect, the present invention relates to a recombinant protein comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V)

In some embodiments, said epitope comprises or consists of an amino acid sequence having at least 85 %, such as 88 %, more preferably at least 95 %, for instance 100 % sequence identity to the amino acid sequence set forth in SEQ ID NO: 63 (GEVPKX₁RFS), wherein X₁ is as defined herein above.

In some embodiments, said epitope comprises or consists of an amino acid sequence set forth in SEQ ID NO: 64 (X₂X₃GEVPKX₁RFSX₄), wherein X₁ is as defined herein above; X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

In some embodiments, said epitope comprises or consists of an amino acid sequence selected from:
- DX₃GEVPKX₁RFSX₄ (SEQ ID NO: 65);
- EX₃GEVPKX₁RFSX₄ (SEQ ID NO: 66);
- X₂KGEVPKX₁RFSX₄ (SEQ ID NO: 67);
- X₂RGEVPKX₁RFSX₄ (SEQ ID NO: 68);
- X₂HGEVPKX₁RFSX₄ (SEQ ID NO: 69);
- X₂X₃GEVPKX₁RFSG (SEQ ID NO: 70);
- X₂X₃GEVPKX₁RFSL (SEQ ID NO: 71);
- X₂X₃GEVPKX₁RFSA (SEQ ID NO: 72);
- X₂X₃GEVPKX₁RFSV (SEQ ID NO: 73);
- X₂X₃GEVPKX₁RFSM (SEQ ID NO: 74);
- X₂X₃GEVPKX₁RFSI (SEQ ID NO: 75);
- X₂X₃GEVPKX₁RFSS (SEQ ID NO: 76);
- X₂X₃GEVPKX₁RFST (SEQ ID NO: 77),
wherein, where applicable, wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V); X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

In some embodiments, said epitope comprises or consists of an amino acid sequence selected from:
- DKGEVPKX₁RFSX₄ (SEQ ID NO: 78);
- DRGEVPKX₁RFSX₄(SEQ ID NO: 79);
- DHGEVPKX₁RFSX₄(SEQ ID NO: 80);
- EKGEVPKX₁RFSX₄ (SEQ ID NO: 81);
- ERGEVPKX₁RFSX₄ (SEQ ID NO: 82);
- EHGEVPKX₁RFSX₄ (SEQ ID NO: 83),
wherein X₁ and X₄ are as defined herein above.

In some embodiments, said epitope comprises an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

In some embodiments, said epitope consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

In various embodiments, said epitope comprises or consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to the amino acid sequence set forth in SEQ ID NO: 1 (DKGEVPKDRFSA).

In various embodiments, said epitope consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to the amino acid sequence set forth in SEQ ID NO: 1 (DKGEVPKDRFSA).

In some embodiments, the recombinant protein of the present invention is a TCR.

In some embodiments, the recombinant protein is a TCR, wherein the epitope, as herein defined, is not present within CDRs or in the surrounding framework region of the TCR.

The invention is now described with respect to some examples which are, however, not be construed as limiting.

### Experiments

### Example 1: Epitope mapping of MDG827 monoclonal antibody

To identify the critical amino acid residues within the epitope set forth in SEQ ID NO:1 that are essential for antibody binding alanine scanning mutagenesis approach was followed. The method involved synthesizing a series of UNI-Tag peptides wherein each amino acid position within the epitope sequence was systematically substituted with alanine (Ala). Subsequently, these peptides underwent analysis using dot blot assays to evaluate their binding with the MDG827 antibody.

The alanine scan shows that aa at position 4(E), 5(V), 6(P), 7(K), & 9(R) are critical amino acid for interacting MDG827 antibody. **(****Fig. 2****)**

### Method:

Briefly, the Alanine mutant peptide (SEQ ID NOs: 18 to 29) was spotted onto a PVDF membrane and allowed to dry. Subsequently, the membrane was blocked with a 5% milk suspension to prevent any non-specific binding before incubating with primary and secondary antibodies. After blocking, the PVDF membrane was washed three times with TBST (Tris-Buffered Saline with 0.05% Tween 20). Then, the blocked membrane was incubated with the primary antibody (MDG827) overnight at 4 degrees Celsius with overhead shaking. The next day, MDG827 was removed, and the membrane was washed three times with TBST before being incubated with HRP-labeled secondary antibody (1:800 dilution, Goat Anti-Human IgG H&L (HRP); Catalog #ab6858) for 60 minutes at room temperature. Subsequently, the secondary antibody was removed, and the membrane was washed twice with TBS (Tris-Buffered Saline) and once with TBST. Finally, the Electrochemiluminescence (ECL) substrate (1:1) was added to the membrane and imaged using chemiluminescence imaging system.

### Example 2: Recombinant monoclonal MDG827 antibody production and functional assay

### Method:

### Production of recombinant antibody

The monoclonal MDG827 light chain and heavy chain were revealed by antibody sequencing (Rapidnovor) and *in silico* transcribed into a nucleotide sequence using the most frequently used codons in the human genome. Since protein sequencing of the antibody does not allow to reveal the leader sequence of the antibody, a known leader sequence was used. The light and heavy nucleotide sequence were individually cloned into the pcDNA^{™}3-TOPO^{™} mammalian expression vector and co-transfected into HEK293 cells to produce the antibody. A mixture of Mirus transfection reagent (Cat # MIR2305, Lot: 02083632) and DMEM III was prepared, to which plasmid containing light chain or heavy chain containing pcDNA^{™}3-TOPO^{™} mammalian expression vector was both added to HEK293 cells. After an incubation period of about 48 hours at 37°C and 5% CO₂, the supernatant was collected for dot-blot assays and cell staining.

### Dot-Blot assay

The Alanine mutant peptides (Uni-TAG-ALA 5 and Uni-Tag-ALA 10) was spotted onto a PVDF membrane and allowed to dry. Subsequently, the membrane was blocked with a 5% milk suspension to prevent any non-specific binding before incubating with primary and secondary antibodies. After blocking, the PVDF membrane was washed three times with TBST (Tris-Buffered Saline with 0.05% Tween 20). Then, the blocked membrane was incubated with the primary antibody (MDG827 hybridoma supernatant or recombinantly produced in HEK293 cells) overnight at 4 degrees Celsius with overhead shaking. The next day, MDG827 was removed, and the membrane was washed three times with TBST before being incubated with HRP-labeled secondary antibody (1:800 dilution, Goat Anti-Human IgG H&L (HRP); Catalog #ab6858) for 60 minutes at room temperature. Subsequently, the secondary antibody was removed, and the membrane was washed twice with TBS (Tris-Buffered Saline) and once with TBST. Finally, the Electrochemiluminescence (ECL) substrate (1:1) was added to the membrane and imaged using chemiluminescence imaging system.

In dot blot analysis, the recombinant monoclonal MDG827 antibody bound to the epitope ALA-10 peptide (positive control; DKGEVPKDRASA set forth in SEQ ID NO: 27), while no binding was observed for the ALA-5 (negative control; DKGEAPKDRFSA set forth in SEQ ID NO: 22). The recombinant monoclonal MDG827 exhibited the exact same binding pattern when compared to MDG827 produced by MDG827 hybridoma cells.

### Cell staining using recombinant produced MDG827

To ensure the expression of rTCR, the first step involved transfecting the gamma viral vector (pES12-6) containing the wildtype PRAME-VLD (T4.8-1-29) or NY-ESO-1 (T11.8-10-17) specific TCR or KRasG12V (T47.8-41-071) or respective specific TCR with Uni-TAG epitope at positions A199 of TRBC were transfected into HEK293FT cells using Mirus transfection reagent (Cat # MIR2305, Lot: 02083632) to generate virus particles. Briefly, a mixture of Mirus transfection reagent and DMEM III was prepared, to which DNA was added for each construct. After an incubation period of about 48 hours at 37°C and 5% CO₂, the virus supernatant (VSN) was collected for transduction.

For transduction, the respective TCR construct VSN was centrifuged to eliminate residual HEK cells, and 1.5 ml/well (24 well plate) of VSN was added to retronectin (Cat # T201, Lot. AGLG003, Takara/Clontech) coated wells. Subsequently, 0.25 Million ieGFP Jurkat-76 -/- CD8+ biosensors cells per/well were seeded onto the well and incubated overnight at 37°C, 5% CO₂. After 72 hours, the transduced cells were used for further analysis (expression analysis via antibody staining.

For analyzing the binding of recombinantly produced anti TRBC-MDG827, the transduced cells were stained with anti-TRBC MDG827 antibody. For anti-TRBC MDG827 antibody staining, briefly, 100k transduced cells were carefully transferred into a well of a 96-well u-bottom plate. The plate was then subjected to centrifugation at 450xg for 5 minutes, and the supernatant was discarded. Following this, the cell pellets were resuspended in 50µl of anti-TRBC MDG827 primary antibody and left to incubate for 30 minutes at 4°C. After the incubation period, 150 µl of FACS buffer was added to all samples, and the plate was centrifuged again at 450xg for 5 minutes, with the supernatant being discarded afterward. The samples underwent two washes with 150 µl of FACS buffer each time, and the supernatant was again discarded after each wash. Subsequently, 50 µl of anti-Rat IgG-PE labelled secondary antibody, prepared at a 1:20 dilution, was added to the samples and incubated with the samples for 30 minutes in the dark at room temperature. After the incubation, 150 µl of FACS buffer was added to all samples, followed by centrifugation at 450xg for 5 minutes and discarding of the supernatant. Finally, 100 µl per sample of FACS buffer with 7AAD, diluted at 1:100, was prepared, and the cell pellets were resuspended in FACS-7AAD buffer for subsequent FACS analysis.

The recombinant monoclonal MDG827 antibody only stained Jurkat 76 -/- CD8+ ieGFP cells expressing the UNI-tag (SEQ ID NO: 1) modified PRAME-VLD (T4.8-1-29) or NYESO1 (T11.8-10-17) specific TCR or KRasG12V (T47.8-41-071), whereas no staining was observed in cells expressing the wildtype non-modified PRAME-VLD (T4.8-1-29) or NY-ESO-1 (T11.8-10-17) specific TCR or KRasG12V (T47.8-41-071) TCR (**Fig. 3**).

The TCR sequences used for cell staining via MDG827 are listed in Table 2 below:

**Table 2: TCR sequences**

| **TCR** | **Amino acid sequence** | **Nucleic acid sequence** |
|---|---|---|
| NYESO-SSL(T11.8-10-17) Alpha Variable & Joining region | SEQ ID NO: 38 | SEQ ID NO: 50 |
| NYESO-SSL(T11.8-10-17) Alpha Constant region | SEQ ID NO: 39 | SEQ ID NO: 51 |
| NYESO-SSL(T11.8-10-17) Beta Variable & Joining region | SEQ ID NO: 40 | SEQ ID NO: 52 |
| NYESO-SSL(T11.8-10-17) Beta Constant region 1 with Uni-Tag | SEQ ID NO: 41 | SEQ ID NO: 53 |
| PRAME-VLD(T4.8-1-29) Alpha Variable & Joining region | SEQ ID NO: 42 | SEQ ID NO: 54 |
| PRAME-VLD(T4.8-1-29) Alpha Constant region | SEQ ID NO: 43 | SEQ ID NO: 55 |
| PRAME-VLD(T4.8-1-29) Beta Variable & Joining region | SEQ ID NO: 44 | SEQ ID NO: 56 |
| PRAME-VLD(T4.8-1-29) Beta Constant region 1 with Uni-Tag | SEQ ID NO: 45 | SEQ ID NO: 57 |
| KRASmut G12V (T47.8-041-071) Alpha Variable & Joining region | SEQ ID NO: 46 | SEQ ID NO: 58 |
| KRASmut G12V (T47.8-041-071) Alpha Constant region | SEQ ID NO: 47 | SEQ ID NO: 59 |
| KRASmut G12V (T47.8-041-071) Beta Variable & Joining region | SEQ ID NO: 48 | SEQ ID NO: 60 |
| KRASmut G12V (T47.8-041-071) Beta Constant region 1 with Uni-Tag | SEQ ID NO: 49 | SEQ ID NO: 61 |

### Specific embodiments:

Embodiment 1: Antibody or binding fragment thereof that binds to a protein, preferably a T cell receptor, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 2: Antibody or binding fragment thereof according to embodiment 1, wherein said epitope comprises or consists of an amino acid sequence having at least 85 %, such as 88 %, more preferably at least 95 %, for instance 100 % sequence identity to the amino acid sequence set forth in SEQ ID NO: 63 (GEVPKX₁RFS), wherein X₁ is as defined herein above.

Embodiment 3: Antibody or binding fragment thereof according to embodiment 1 or 2, wherein said epitope comprises or consists of an amino acid sequence set forth in SEQ ID NO: 64 (X₂X₃GEVPKX₁RFSX₄), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V); X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

Embodiment 4: Antibody or binding fragment thereof according to any of the preceding embodiments, wherein said epitope comprises or consists of an amino acid sequence selected from:
- DX₃GEVPKX₁RFSX₄ (SEQ ID NO: 65);
- EX₃GEVPKX₁RFSX₄ (SEQ ID NO: 66);
- X₂KGEVPKX₁RFSX₄ (SEQ ID NO: 67);
- X₂RGEVPKX₁RFSX₄ (SEQ ID NO: 68);
- X₂HGEVPKX₁RFSX₄ (SEQ ID NO: 69);
- X₂X₃GEVPKX₁RFSG (SEQ ID NO: 70);
- X₂X₃GEVPKX₁RFSL (SEQ ID NO: 71);
- X₂X₃GEVPKX₁RFSA (SEQ ID NO: 72);
- X₂X₃GEVPKX₁RFSV (SEQ ID NO: 73);
- X₂X₃GEVPKX₁RFSM (SEQ ID NO: 74);
- X₂X₃GEVPKX₁RFSI (SEQ ID NO: 75);
- X₂X₃GEVPKX₁RFSS (SEQ ID NO: 76);
- X₂X₃GEVPKX₁RFST (SEQ ID NO: 77),
wherein, where applicable, wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V); X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

Embodiment 5: Antibody or binding fragment thereof according to any of the preceding embodiments, wherein said epitope comprises or consists of an amino acid sequence selected from:
- DKGEVPKX₁RFSX₄ (SEQ ID NO: 78);
- DRGEVPKX₁RFSX₄(SEQ ID NO: 79);
- DHGEVPKX₁RFSX₄(SEQ ID NO: 80);
- EKGEVPKX₁RFSX₄ (SEQ ID NO: 81);
- ERGEVPKX₁RFSX₄ (SEQ ID NO: 82);
- EHGEVPKX₁RFSX₄ (SEQ ID NO: 83),
wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V), and X₄ is selected from glycine (G), leucine (L), alanine (A), valine (V), methionine (M), isoleucine (I), serine (S), and threonine (T).

Embodiment 6: Antibody or binding fragment thereof according to any of the preceding embodiments, wherein said epitope comprises or consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 7: Antibody or binding fragment thereof according to any of the preceding embodiments, wherein said epitope consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 8: Antibody or binding fragment thereof according to any of the preceding embodiments, wherein the antibody or binding fragment thereof is characterized in comprising a light chain variable region comprising at least a CDR1 set forth in SEQ ID NO: 3 or sequences at least 80% identical thereto, a CDR2 set forth in SEQ ID NO: 4 or sequences at least 80% identical thereto, and/or a CDR3 set forth in SEQ ID NO: 5 or sequences at least 80% identical thereto.

Embodiment 9: Antibody or binding fragment thereof according to any of the preceding embodiments, wherein the antibody or binding fragment thereof is characterized in comprising a light chain variable region comprising at least a CDR1 set forth in SEQ ID NO: 3 or sequences at least 80% identical thereto, a CDR2 set forth in SEQ ID NO: 4 or sequences at least 80% identical thereto, and a CDR3 set forth in SEQ ID NO: 5 or sequences at least 80% identical thereto.

Embodiment 10: Antibody or binding fragment thereof according to any of the preceding embodiments, wherein the antibody or binding fragment thereof is characterized in comprising a heavy chain variable region comprising at least a CDR1 set forth in SEQ ID NO: 7 or sequences at least 80% identical thereto, a CDR2 set forth in SEQ ID NO: 8 or sequences at least 80% identical thereto, and/or a CDR3 set forth in SEQ ID NO: 9 or sequences at least 80% identical thereto.

Embodiment 11: Antibody or binding fragment thereof according to any of the preceding embodiments, wherein the antibody or binding fragment thereof is characterized in comprising a heavy chain variable region comprising at least a CDR1 set forth in SEQ ID NO: 7 or sequences at least 80% identical thereto, a CDR2 set forth in SEQ ID NO: 8 or sequences at least 80% identical thereto, and a CDR3 set forth in SEQ ID NO: 9 or sequences at least 80% identical thereto.

Embodiment 12: Antibody or binding fragment thereof according to any of the preceding embodiments, wherein the antibody or binding fragment thereof is characterized in that the light chain variable region comprises at least a CDR1 set forth in SEQ ID NO: 3 or sequences at least 80% identical thereto, a CDR2 set forth in SEQ ID NO: 4 or sequences at least 80% identical thereto, and/or a CDR3 set forth in SEQ ID NO: 5 or sequences at least 80% identical thereto; and/or in that the heavy chain variable region comprises at least a CDR1 set forth in SEQ ID NO: 7 or sequences at least 80% identical thereto, a CDR2 set forth in SEQ ID NO: 8 or sequences at least 80% identical thereto, and/or a CDR3 set forth in SEQ ID NO: 9 or sequences at least 80% identical thereto.

Embodiment 13: Antibody or binding fragment thereof according to any of the preceding embodiments, wherein the antibody or binding fragment thereof is characterized in that the light chain variable region comprises at least a CDR1 set forth in SEQ ID NO: 3 or sequences at least 80% identical thereto, a CDR2 set forth in SEQ ID NO: 4 or sequences at least 80% identical thereto, and a CDR3 set forth in SEQ ID NO: 5 or sequences at least 80% identical thereto; and/or in that the heavy chain variable region comprises at least a CDR1 set forth in SEQ ID NO: 7 or sequences at least 80% identical thereto, a CDR2 set forth in SEQ ID NO: 8 or sequences at least 80% identical thereto, and a CDR3 set forth in SEQ ID NO: 9 or sequences at least 80% identical thereto.

Embodiment 14: Antibody or binding fragment thereof according to any of the preceding embodiments, wherein the antibody or binding fragment thereof is characterized in that the light chain variable region comprises at a CDR1 set forth in SEQ ID NO: 3, a CDR2 set forth in SEQ ID NO:, and a CDR3 set forth in SEQ ID NO: 5; and in that the heavy chain variable region comprises at least a CDR1 set forth in SEQ ID NO: 7, a CDR2 set forth in SEQ ID NO: 8, and a CDR3 set forth in SEQ ID NO: 9.

Embodiment 15: Antibody or binding fragment thereof according to the preceding embodiments wherein the antibody or binding fragment thereof is capable of depleting a subpopulation of T cells expressing a fraction of TCR Vα chains comprising at least two different TCR Vα chains or is capable of depleting a subpopulation of T cells expressing a fraction of TCR Vβ chains comprising at least two different TCR Vβ chains.

Embodiment 16: Antibody or binding fragment thereof according to any one of the preceding embodiments, wherein the antibody or binding fragment thereof is characterized in that the light chain variable region comprises at least a CDR1 set forth in SEQ ID NO: 3 or sequences at least 80% identical thereto, a CDR2 set forth in SEQ ID NO: 4 or sequences at least 80% identical thereto, and a CDR3 set forth in SEQ ID NO: 5 or sequences at least 80% identical thereto; and in that the heavy chain variable region comprises at least a CDR1 set forth in SEQ ID NO: 7 or sequences at least 80% identical thereto, a CDR2 set forth in SEQ ID NO: 8 or sequences at least 80% identical thereto, and a CDR3 set forth in SEQ ID NO: 9 or sequences at least 80% identical thereto.

Embodiment 17: Antibody or binding fragment thereof according to any one of the preceding embodiments, wherein the antibody or binding fragment thereof is characterized in comprising a light chain variable region comprising at least a CDR1 encoded by the nucleotide sequence set forth in SEQ ID NO: 11 or sequences at least 80% identical thereto, and/or a CDR2 encoded by the nucleotide sequence set forth in SEQ ID NO: 12 or sequences at least 80% identical thereto, and/or a CDR3 encoded by the nucleotide sequence set forth in SEQ ID NO: 13 or sequences at least 80% identical thereto.

Embodiment 18: Antibody or binding fragment thereof according to any of the preceding embodiments, wherein the antibody or binding fragment thereof is characterized in comprising a light chain variable region comprising at least a CDR1 encoded by the nucleotide sequence set forth in SEQ ID NO: 11 or sequences at least 80% identical thereto, a CDR2 encoded by the nucleotide sequence set forth in SEQ ID NO: 12 or sequences at least 80% identical thereto, and a CDR3 encoded by the nucleotide sequence set forth in SEQ ID NO: 13 or sequences at least 80% identical thereto.

Embodiment 19: Antibody or binding fragment thereof according to any of the preceding embodiments, wherein the antibody or binding fragment thereof is characterized in comprising a heavy chain variable region comprising at least a CDR1 encoded by the nucleotide sequence set forth in SEQ ID NO: 15 or sequences at least 80% identical thereto, and/or a CDR2 encoded by the nucleotide sequence set forth in SEQ ID NO: 16 or sequences at least 80% identical thereto, and/or a CDR3 encoded by the nucleotide sequence set forth in SEQ ID NO: 17 or sequences at least 80% identical thereto.

Embodiment 20: Antibody or binding fragment thereof according to any of the preceding embodiments, wherein the antibody or binding fragment thereof is characterized in comprising a heavy chain variable region comprising at least a CDR1 encoded by the nucleotide sequence set forth in SEQ ID NO: 15 or sequences at least 80% identical thereto, a CDR2 encoded by the nucleotide sequence set forth in SEQ ID NO: 16 or sequences at least 80% identical thereto, and a CDR3 encoded by the nucleotide sequence set forth in SEQ ID NO: 17 or sequences at least 80% identical thereto.

Embodiment 21: Antibody or binding fragment thereof according to any of the preceding embodiments, wherein the antibody or binding fragment thereof is characterized in that the light chain variable region comprises at least a CDR1 encoded by the nucleotide sequence set forth in SEQ ID NO: 11 or sequences at least 80% identical thereto, a CDR2 encoded by the nucleotide sequence set forth in SEQ ID NO: 12 or sequences at least 80% identical thereto, and/or a CDR3 encoded by the nucleotide sequence set forth in SEQ ID NO: 13 or sequences at least 80% identical thereto; and/or in that the heavy chain variable region comprises at least a CDR1 encoded by the nucleotide sequence set forth in SEQ ID NO: 15 or sequences at least 80% identical thereto, a CDR2 encoded by the nucleotide sequence set forth in SEQ ID NO: 16 or sequences at least 80% identical thereto, and/or a CDR3 encoded by the nucleotide sequence set forth in SEQ ID NO: 17 or sequences at least 80% identical thereto.

Embodiment 22: Antibody or binding fragment thereof according to any of the preceding embodiments, wherein the antibody or binding fragment thereof is characterized in that the light chain variable region comprises at least a CDR1 encoded by the nucleotide sequence set forth in SEQ ID NO: 11 or sequences at least 80% identical thereto, a CDR2 encoded by the nucleotide sequence set forth in SEQ ID NO: 12 or sequences at least 80% identical thereto, and a CDR3 encoded by the nucleotide sequence set forth in SEQ ID NO: 13 or sequences at least 80% identical thereto; and/or in that the heavy chain variable region comprises at least a CDR1 encoded by the nucleotide sequence set forth in SEQ ID NO: 15 or sequences at least 80% identical thereto, a CDR2 encoded by the nucleotide sequence set forth in SEQ ID NO: 16 or sequences at least 80% identical thereto, and a CDR3 encoded by the nucleotide sequence set forth in SEQ ID NO: 17 or sequences at least 80% identical thereto.

Embodiment 23: Antibody or binding fragment thereof according to any of the preceding embodiments, wherein the antibody or binding fragment thereof is characterized in that the light chain variable region comprises at least a CDR1 encoded by the nucleotide sequence set forth in SEQ ID NO: 11 or sequences at least 80% identical thereto, a CDR2 encoded by the nucleotide sequence set forth in SEQ ID NO: 12 or sequences at least 80% identical thereto, and a CDR3 encoded by the nucleotide sequence set forth in SEQ ID NO: 13 or sequences at least 80% identical thereto; and in that the heavy chain variable region comprises at least a CDR1 encoded by the nucleotide sequence set forth in SEQ ID NO: 15 or sequences at least 80% identical thereto, a CDR2 encoded by the nucleotide sequence set forth in SEQ ID NO: 16 or sequences at least 80% identical thereto, and a CDR3 encoded by the nucleotide sequence set forth in SEQ ID NO: 17 or sequences at least 80% identical thereto.

Embodiment 24: Antibody or binding fragment thereof according to any of the preceding embodiments, wherein the antibody or binding fragment thereof is capable of tagging a protein, preferably a TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 25: Antibody or binding fragment thereof according to any of the preceding embodiments, said antibody being a monoclonal antibody, preferably a monoclonal chimeric, murine, humanized or human antibody.

Embodiment 26: Antibody or binding fragment thereof according to any of the preceding embodiments, said antibody being selected from antibody subtypes of the IgG or IgM class.

Embodiment 27: Antibody or binding fragment thereof according to any of the preceding embodiments, said antibody being selected from the IgG2a subtype, preferably the rat IgG2a subtype, particularly comprising a kappa light chain.

Embodiment 28: Use of an antibody or binding fragment thereof according to any one of embodiments 1 to 27 for tagging a protein, preferably a TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 29: Use of an antibody or binding fragment thereof according to any one of embodiments 1 to 27 for ex *vivo* tagging a protein, preferably a TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 30: Antibody or binding fragment thereof according to any one of embodiments 1 to 27 for use as a medicament.

Embodiment 31: Antibody or binding fragment thereof according to any one of embodiments 1 to 27 for use as a screening agent.

Embodiment 32: Antibody or binding fragment thereof according to any one of embodiments 1 to 27 for use in the therapy of malignancies, preferably for use in the treatment of cancer, viral diseases, or autoimmunity.

Embodiment 33: Recombinant protein comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 34: Recombinant protein according to embodiment 33, wherein said epitope comprises or consists of an amino acid sequence having at least 85 %, such as 88 %, more preferably at least 95 %, for instance 100 % sequence identity to the amino acid sequence set forth in SEQ ID NO: 63 (GEVPKX₁RFS), wherein X₁ is as defined herein above.

Embodiment 35: Recombinant protein according to according to embodiment 33 or 34, wherein said epitope comprises or consists of an amino acid sequence set forth in SEQ ID NO: 64 (X₂X₃GEVPKX₁RFSX₄), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V); X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

Embodiment 36: Recombinant protein according to embodiments 33 to 35, wherein said epitope comprises or consists of an amino acid sequence selected from:
- DX₃GEVPKX₁RFSX₄ (SEQ ID NO: 65);
- EX₃GEVPKX₁RFSX₄ (SEQ ID NO: 66);
- X₂KGEVPKX₁RFSX₄ (SEQ ID NO: 67);
- X₂RGEVPKX₁RFSX₄ (SEQ ID NO: 68);
- X₂HGEVPKX₁RFSX₄ (SEQ ID NO: 69);
- X₂X₃GEVPKX₁RFSG (SEQ ID NO: 70);
- X₂X₃GEVPKX₁RFSL (SEQ ID NO: 71);
- X₂X₃GEVPKX₁RFSA (SEQ ID NO: 72);
- X₂X₃GEVPKX₁RFSV (SEQ ID NO: 73);
- X₂X₃GEVPKX₁RFSM (SEQ ID NO: 74);
- X₂X₃GEVPKX₁RFSI (SEQ ID NO: 75);
- X₂X₃GEVPKX₁RFSS (SEQ ID NO: 76);
- X₂X₃GEVPKX₁RFST (SEQ ID NO: 77),
wherein, where applicable, wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V); X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

Embodiment 37: Recombinant protein according to embodiments 33 to 36, wherein said epitope comprises or consists of an amino acid sequence selected from:
- DKGEVPKX₁RFSX₄ (SEQ ID NO: 78);
- DRGEVPKX₁RFSX₄(SEQ ID NO: 79);
- DHGEVPKX₁RFSX₄(SEQ ID NO: 80);
- EKGEVPKX₁RFSX₄ (SEQ ID NO: 81);
- ERGEVPKX₁RFSX₄ (SEQ ID NO: 82);
- EHGEVPKX₁RFSX₄ (SEQ ID NO: 83),
wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V), and X₄ is selected from glycine (G), leucine (L), alanine (A), valine (V), methionine (M), isoleucine (I), serine (S), and threonine (T).

Embodiment 38: Recombinant protein according to embodiments 33 to 37, wherein said epitope comprises or consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 39: Recombinant protein according to embodiments 33 to 38, wherein said epitope consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

Embodiment 40: Recombinant protein according to embodiments 33 to 39, wherein the recombinant protein is a TCR, wherein optionally the epitope is not present within CDRs or in the surrounding framework region of the TCR.

Embodiment 41: Pharmaceutical composition comprising an antibody or binding fragment thereof according to any one of the embodiments 1 to 27, or comprising a recombinant protein according to embodiments 33 to 40.

Embodiment 42: Pharmaceutical composition according to embodiment 41, further comprising at least one pharmaceutically acceptable agent, preferably carrier.

## Claims

1. Antibody or binding fragment thereof that binds to a protein, preferably a T cell receptor, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

2. Antibody or binding fragment thereof according to claim 1, wherein the epitope comprises or consists of an amino acid sequence having at least 85 %, such as 88 %, more preferably at least 95 %, for instance 100 % sequence identity to the amino acid sequence set forth in SEQ ID NO: 63 (GEVPKX₁RFS), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

3. Antibody or binding fragment thereof according to any one of the preceding claims, wherein the epitope comprises or consists of an amino acid sequence set forth in SEQ ID NO: 64 (X₂X₃GEVPKX₁RFSX₄), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V); X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).

4. Antibody or binding fragment thereof according to any one of the preceding claims, wherein the epitope comprises or consists of an amino acid sequence having at least 65 %, such as 66 %, at least 75 %, such as 77 %, preferably at least 80 %, such as 83 %, more preferably at least 90 %, such as 91 %, 88 %, or 100 % sequence identity to an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

5. Antibody or binding fragment thereof according to any one of the preceding claims, wherein the antibody or binding fragment thereof belongs to the rat IgG2a subtype, preferably comprising a kappa light chain.

6. Antibody or binding fragment thereof according to any one of the preceding claims, comprising a light chain variable region and/or a heavy chain variable region, wherein the light chain variable region comprises at least a CDR1 set forth in SEQ ID NO: 3 or sequences at least 80% identical thereto, a CDR2 set forth in SEQ ID NO: 4 or sequences at least 80% identical thereto, and/or a CDR3 set forth in SEQ ID NO: 5 or sequences at least 80% identical thereto; and/or wherein the heavy chain variable region comprises at least a CDR1 set forth in SEQ ID NO: 7 or sequences at least 80% identical thereto, a CDR2 set forth in SEQ ID NO: 8 or sequences at least 80% identical thereto, and/or a CDR3 set forth in SEQ ID NO: 9 or sequences at least 80% identical thereto.

7. Antibody or binding fragment thereof according to any one of the preceding claims, comprising a light chain variable region and a heavy chain variable region, wherein the light chain variable region comprises at least a CDR1 set forth in SEQ ID NO: 3, a CDR2 set forth in SEQ ID NO: 4, a CDR3 set forth in SEQ ID NO: 5; and wherein the heavy chain variable region comprises at least a CDR1 set forth in SEQ ID NO: 7, a CDR2 set forth in SEQ ID NO: 8, and/or a CDR3 set forth in SEQ ID NO: 9.

8. Antibody or binding fragment thereof according to any one of the preceding claims, comprising a light chain comprising or consisting of SEQ ID NO: 2 or sequences at least 80% identical thereto and/or a heavy chain comprising or consisting of SEQ ID NO: 6 or sequences at least 80% identical thereto.

9. Antibody or binding fragment thereof according to claim 8, wherein the antibody or binding fragment thereof comprises a light chain comprising or consisting of SEQ ID NO: 2 and/or a heavy chain comprising or consisting of SEQ ID NO: 6.

10. Pharmaceutical composition comprising at least one antibody or binding fragment thereof according to any one of claims 1 to 9.

11. The pharmaceutical composition according to claim 10, further comprising at least one pharmaceutically acceptable carrier.

12. Antibody or binding fragment thereof according to any one of claims 1 to 9 for use as a medicament or for use as a screening agent.

13. Antibody or binding fragment thereof according to any one of claims 1 to 9 for use in the therapy of malignancies, preferably for use in the treatment of cancer, viral diseases, or autoimmunity.

14. Use of an antibody or binding fragment thereof for tagging a protein, preferably a TCR, comprising an epitope comprising or consisting of an amino acid sequence having at least 80 %, such as 83 %, preferably at least 90 %, for instance 100 % sequence identity to the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

15. Recombinant protein, **characterized in** comprising an epitope comprising or consisting of the amino acid sequence set forth in SEQ ID NO: 62 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).

16. The recombinant protein according to claim 15, wherein said epitope comprises or consists of an amino acid sequence selected from the group consisting of SEQ ID NO: 84 (DKGEVPKX₁RFSA), SEQ ID NO: 85 (KGEVPKX₁RFSA), SEQ ID NO: 86 (GEVPKX₁RFSA), SEQ ID NO: 87 (GEVPKX₁RFS), SEQ ID NO: 88 (DKGEVPKX₁RFS), and SEQ ID NO: 89 (KGEVPKX₁RF), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).
